(19)

**Europäisches Patentamt**
**European Patent Office**
**Office européen des brevets**

(11) **EP 4 085 864 B1**

(12) **EUROPEAN PATENT SPECIFICATION**

(45) Date of publication and mention
of the grant of the patent:
**08.05.2024 Bulletin 2024/19**

(21) Application number: **20908726.1**

(22) Date of filing: **30.11.2020**

(51) International Patent Classification (IPC):
**A61B 34/37** (2016.01)    **A61B 34/30** (2016.01)
**A61B 17/00** (2006.01)    **H02K 7/10** (2006.01)

(52) Cooperative Patent Classification (CPC):
**A61B 34/37;** A61B 2090/034

(86) International application number:
**PCT/CN2020/132948**

(87) International publication number:
**WO 2021/135786 (08.07.2021 Gazette 2021/27)**

(54) **TRANSMISSION ASSEMBLY, DRIVE BOX, SURGICAL INSTRUMENT SYSTEM, AND ROBOT SYSTEM**

ÜBERTRAGUNGSANORDNUNG, ANTRIEBSKASTEN, CHIRURGISCHES INSTRUMENTENSYSTEM UND ROBOTERSYSTEM

BLOC DE TRANSMISSION, BOÎTIER D'ENTRAÎNEMENT, SYSTÈME D'INSTRUMENT CHIRURGICAL, ET SYSTÈME DE ROBOT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.12.2019 CN 201911398468**

(43) Date of publication of application:
**09.11.2022 Bulletin 2022/45**

(73) Proprietor: **Shanghai Microport Medbot (Group) Co., Ltd.**
**Shanghai 201203 (CN)**

(72) Inventors:
• **HE, Yuyuan**
**Shanghai 201203 (CN)**

• **HE, Chao**
Shanghai 201203 (CN)
• **TANG, Zhonghua**
Shanghai 201203 (CN)

(74) Representative: **Patentship**
**Patentanwaltsgesellschaft mbH**
**Elsenheimerstraße 65**
**80687 München (DE)**

(56) References cited:
EP-A1- 3 033 030        EP-A1- 3 714 829
EP-B1- 3 033 030        CN-A- 102 256 550
CN-A- 105 997 254       CN-A- 110 464 467
CN-A- 111 000 636       US-A- 4 979 949
US-A1- 2010 170 519

## Description

## TECHNICAL FIELD

The present invention relates to the field of medical instruments, and in particular, to a transmission assembly, a drive box, a surgical instrument system, and a surgical robot system.

## BACKGROUND

In recent years, with the increasing application and development of robotics, in particular of computing technology, more and more importance is being attached to the role of surgical robots in clinical practice. Among such surgical robots, minimally invasive surgical robot can relieve surgeons from strenuous physical labor while achieving precision surgery, and result in less trauma and bleeding, reduced postoperative infections and faster postoperative recovery, of patients. Operating accuracy of a minimally invasive surgical robot, which largely depends on its mechanical structural design, is a key metric for assessing the robot's performance.

During minimally invasive surgery, surgical instruments are used depending on actual circumstances, and driving these surgical instruments requires the use of transmission assemblies capable of both torque transmission and axial displacement compensation. Existing surgical robots employ various transmission assemblies, which, however, all suffer from low transmission accuracy, poor loading conditions of transmission parts and other problems.

Chinese Patent Application Publication No. CN107595394A discloses a mechanism for quick-swap of surgical instruments, in which a cavity underlies a mount recess, and a support shaft is fixed in the cavity so as to be perpendicular to a bottom surface of the mount recess. A drive wheel is coaxially disposed over the support shaft so as to be movable up and down. A spring is provided between the drive wheel and the bottom of the cavity. A circumferential limiting location for the drive wheel is near a centerline of the shaft. This mechanism has the following problems: 1) it is inadequately accurate because, at a given clearance, the closer the circumferential limiting location is to the centerline of the shaft, the lower transmission accuracy will be (according to Angular Deviation ($\Delta\alpha$) $\approx$ Clearance ($\Delta$L)/r, where r represents a distance from the circumferential limiting location to a center of rotation); 2) a force loading condition of the transmission shaft is poor (Force on Each Mating Surface (F) ~ 0.5 * Transmitted Torque (M)/r) and transmission parts are subject to heavy stress which may shorten their service lives, i.e., in order to transmit a given torque, the shorter the distances from the force-loaded surfaces for torque transmission to the center of rotation is, the greater forces the surfaces will be subject to, the more limited the force-loaded areas will be, and the more heavily the parts will be stressed; 3) utilization of a space in its axial dimension that is dependent on a minimum compressed size of the spring is not high enough, which affects the utilization of a distal space of the mechanical arm and weight reduction thereof; 4) its transmission efficiency is not ideal, as it is generally believed that transmission efficiency of worm gears is lower than that of gear transmissions, chain transmissions, belt transmissions and cable transmissions, and insufficient efficiency means that under a given loading condition, a motor capable of providing higher out power is required to achieve the same function; 5) it does not allow for convenient installation and maintenance as it employs an additional axial limiting stopper which affects the convenience of installation and maintenance; and 6) it is incapable of preventing the ingress of dust and foreign matter, and without such dust prevention means, dust tends to enter clearances created during axial movement in the course of long-term use, affecting the smoothness of axial movement.

Chinese Patent Application Publication No. CN101340852A discloses a robotic manipulator including an instrument interface. The instrument interface includes a spring-loaded input for providing axial load and torque to a sterile adaptor. This spring-loaded input includes a spring, radial ball bearings, an output pulley, a linear ball spline slide unit, a ball spline shaft, a screw and an input bar including bosses. Likewise, the ball spline for providing circumferential limitation is also located close to a centerline of the shaft. This instrument interface has the following problems: 1) it is inadequately accurate because, at a given clearance, the closer circumferential limitation is accomplished to the centerline of the shaft, the lower transmission accuracy will be (according to Angular Deviation ($\Delta\alpha$) $\approx$ Clearance ($\Delta$L)/r, where r represents a distance from the circumferential limiting location to a center of rotation); 2) a force loading condition of the transmission shaft is poor and the transmission parts are subject to heavy stress which may shorten their service lives, i.e., in order to transmit a given torque, the shorter the distances from the force-loaded surfaces for torque transmission to the center of rotation is, the greater forces the surfaces will be subject to (Force on Each Mating Surface (F) ~ 0.5 * Transmitted Torque (M)/r), the more limited the force-loaded areas will be, and the more heavily the parts will be stressed; 3) utilization of a space in its axial dimension that is dependent on a minimum compressed size of the spring is not high enough, which affects the utilization of a distal space of the mechanical arm and weight reduction thereof, and as a standard part, the ball spline has a relatively large axial dimension; and 4) its structure does not allow easy, quick and secure connection with conventional drive shafts (motor shafts, reducer shafts).

EP 3 714 829 A1 discloses an adaptor including a drive transmission member to transmits a driving force from a driving member of a drive part to a driven member of a surgical instrument. EP 3 033 030 A1 discloses a surgical system including a surgical instrument that is sensitive to backlash that would adversely affect the transmission of controlled torque and position to the surgical instrument. US 2010/170519 A1 discloses a robotic surgical system configured to perform minimally invasive surgical procedures.

## SUMMARY OF THE INVENTION

The invention is set out in the appended set of claims.

In view of one or more of the above problems, it is an aim of the present invention to provide a transmission assembly with increased transmission accuracy and an improved loading condition of its transmission components, as well as a drive box, a surgical instrument system and a robot system.

In pursuit of the above aim, the present invention provides a transmission assembly, including:

an input base having a first end and an opposing second end, the first end configured for connection with a drive mechanism;

a guide transmission disposed on an outer circumferential surface of the input base;

an output base having a third end and an opposing fourth end, the third end located closer to the first end than the fourth end;

an axial energy storage member configured to store energy during movement of the input base and the output base toward each other under the action of an external force and to release, upon removal of the external force, the energy to drive movement of the input base and the output base away from each other;

an axial limiting assembly configured to limit a maximum displacement of the input base and the output base from each other,

wherein the output base is configured to cooperate with the guide transmission so that when the input base is driven by the drive mechanism to rotate, the guide transmission causes the output base to rotate in synchronization with the input base.

The third end of the output base is an open end, wherein the output base defines a cavity in which the second end of the input base is inserted.

The guide transmission is defined by an external extension of the outer circumferential surface of the input base, wherein the cavity is provided in its inner wall with a mating internal groove that matches the guide transmission.

Optionally, the mating internal groove and the guide transmission may cooperate with each other to define a torque transmission surface including a proximal end and a distal end, the proximal end of the torque transmission surface spaced apart from a center of the output base by a distance $r_t$ satisfying the relationship $0.3R_S \leq r_t < R_S$, the distal end of the torque transmission surface spaced apart from the center of the output base by a distance $r_m$ satisfying the relationship $r_t < r_m \leq R_S$, wherein $(r_m - r_t) \in [0.03R_S, 0.7R_S]$, where $R_S$ represents a distance between the center and the most distant boundary of the output base from the center.

Optionally, the transmission assembly may further include a rolling member disposed between the guide transmission and the mating internal groove in order to enable torque transmission between the guide transmission and the mating internal groove.

Optionally, the rolling member may include rolling elements and a guide channel, which are separately provided on the guide transmission and the mating internal groove, the rolling elements including at least one roller and a roller cage, each roller provided in the roller cage such as to be rollable in the guide channel, the roller cage configured to keep each roller at a constant position relative to the roller cage, the guide channel arranged along an axis of the transmission assembly.

Optionally, the guide transmission may be connected to the outer circumferential surface of the input base by a cantilever, wherein the output base is provided on its outer wall with a mating external groove that matches the guide transmission.

Optionally, the mating external groove and the guide transmission may cooperate with each other to define a torque transmission surface including a proximal end and a distal end, the proximal end of the torque transmission surface spaced apart from a center of the output base by a distance $r_t$ satisfying the relationship $0.3R_S \leq r_t < R_S$, the distal end of the torque transmission surface spaced apart from the center of the output base by a distance $r_m$ satisfying the relationship $r_t < r_m \leq R_S$, and wherein $(r_m - r_t) \in [0.03R_S, 0.7R_S]$, where $R_S$ represents a distance between the center and the most distant boundary of the output base from the center.

Optionally, the transmission assembly may further include a rolling member disposed between the guide transmission and the mating external groove in order to enable torque transmission between the guide transmission and the mating external groove.

Optionally, the rolling member may include rolling elements and a guide channel, which are separately provided on the guide transmission and the mating external groove, the rolling elements including at least one roller and a roller cage, each roller provided in the roller cage such as to rollable in the guide channel, the roller cage configured to keep each roller at a constant position relative to the roller cage, the guide channel arranged along an axis of the transmission assembly.

Optionally, the input base may be provided at the first end with a mounting hole for enabling the input base to be coupled to the drive mechanism.

Optionally, the input base may be also provided with a locking hole which cooperates with a locking element to secure the drive mechanism to the input base.

Optionally, the axial energy storage member may include at least one elastic element brought into abutment against the input base at one end and against the output base at the other end.

Optionally, the axial energy storage member may include two mutually repulsive magnetic elements, one of which is provided on the input base, and the other is provided on the output base.

Optionally, the axial limiting assembly may include a first axial limiting sub-assembly including a first stopper and a first limiting stopper, the first stopper provided on an end face of the input base at the second end, the first limiting stopper provided on the inner wall of the cavity,

wherein upon the first stopper coming into interaction with the first limiting stopper, the output base is stopped from further axially moving away from the input base.

Optionally, the first stopper may be a first screw provided on the end face of the input base at the second end, wherein the first limiting stopper is provided on an inner side wall of the output base so as to radially extend inwardly from the inner side wall.

Optionally, the input base may be provided in the end face at the second end with a first screw hole, wherein the output base is provided at the fourth end with an axially extending through mounting hole that is coaxial with the first screw hole, the first screw inserted through the through mounting hole and threadedly connected to the first screw hole.

Optionally, the output base may be further provided in its outer wall at the fourth end with a recess in which the through mounting hole is provided, wherein the transmission assembly further includes a cover plate configured to cover the recess to prevent the ingress of dust.

Optionally, the axial limiting assembly may include a second axial limiting sub-assembly including a second stopper and a second limiting stopper, the second stopper provided on the outer circumferential surface of the input base proximal to the first end, the second limiting stopper provided at an end face of the output base at the third end, and wherein upon the second stopper coming into interaction with the second limiting stopper, the output base is stopped from further axially moving toward the input base.

The axial limiting assembly includes a first axial limiting sub-assembly including a third limiting stopper and a third stopper, the third limiting stopper provided on the outer circumferential surface of the input base, the third stopper provided on an end face of the output base at the third end, wherein the output base is provided in the cavity with a receptacle channel which matches the third limiting stopper so as to allow axial movement thereof, and

wherein upon the third stopper coming into interaction with the third limiting stopper, the output base is stopped from further axially moving away from the input base.

Optionally, the third stopper may be a second screw, wherein the third limiting stopper is provided on the outer circumferential surface of the input base proximal to the second end such as to radially extend outwardly from the input base.

Optionally, the axial limiting assembly may include a second axial limiting sub-assembly including a fourth stopper and a fourth limiting stopper, the fourth stopper provided on an end face of the input base at the second end, the fourth limiting stopper provided on an inner wall of the output base at the fourth end, and

wherein upon the fourth stopper coming into interaction with the fourth limiting stopper, the output base is stopped from further axially moving toward the input base.

Optionally, the third stopper may include a limiting end and a connecting end, with the output base being provided with a connecting hole configured for threaded connection with the connecting end, wherein when projected axially, the sum of a diameter of the connecting hole and a preset safe thickness for a threaded wall in the connecting hole is 0.6-0.8 times a diameter of the limiting end.

Optionally, the mating internal groove may define a line of symmetry,

wherein when projected axially, the transmission assembly defines a first radial line and a second radial line, the first radial line corresponding to the line of symmetry of the mating internal groove, the second radial line being a straight line passing through a center of the transmission assembly and a start position of the third limiting stopper on the outer circumferential surface of the input base at the second end, and

wherein the start position of the third limiting stopper is within the following range:

$$\beta \in [\pi - \arcsin\frac{1.6d_2}{d_1 - d_2} - \arcsin\frac{d_2 + w}{d_1 - d_2}, \pi - \arcsin\frac{1.1d_2}{d_1 - d_2} - \arcsin\frac{d_2 + w}{d_1 - d_2}] \quad,$$

where w is the sum of a width of the mating internal groove and widths of its two side walls, $\beta$ is an angle required for the first radial line to clockwise rotate to the second radial line, $d_1$ is a diameter of a circumscribed circle of the transmission assembly, and $d_2$ is a diameter of the limiting end.

Optionally, when projected axially, a distance from any point on the third limiting stopper to a center of the limiting end may be greater than 0.3 times the diameter of the limiting end.

Optionally, the input base may be provided with a locking hole configured for connection with a drive mechanism, wherein when projected axially, the locking hole defines an imaginary rectangle and the transmission assembly further defines a fourth radial line passing through the center of the transmission assembly and a middle point of a short side of the imaginary rectangle, and

wherein a relative position of the locking hole is within the following range:

$$\gamma \in [\arctan\frac{L_1}{L_2} + \arcsin\frac{w}{\sqrt{L_1^2 + L_2^2}}, \pi - \arcsin\frac{1.1d_2}{d_1 - d_2} - \arcsin\frac{d_2 + w}{d_1 - d_2} - \arctan\frac{L_1}{L_2}] \quad,$$

where $\gamma$ is an angle required for the first radial line to clockwise rotate to the fourth radial line, $L_1$ is a length of the short side of the imaginary rectangle, and $L_2$ is a length of a long side of the imaginary rectangle.

In pursuit of the above aim, the present invention also provides a drive box including:

a housing provided therein with at least one output hole;

at least one transmission assembly according to any of the preceding paragraphs ; and

at least one drive mechanism disposed in the housing so as to extend out from the output hole and come into connection with the first end of the input base in the transmission assembly.

Optionally, the input base may be provided at the first end with a mounting hole configured to enable the connection of the input base and the drive mechanism, wherein the input base is provided with a first locking hole section and the drive mechanism is provided with a second locking hole section, and wherein a locking element cooperates with the first and second locking hole sections to secure the input base to the drive mechanism.

Optionally, the drive mechanism may have a profiled output end portion, wherein the mounting hole in the transmission assembly has a shape matching the profiled output end portion.

In pursuit of the above aim, the present invention also provides a surgical instrument system including:

a surgical instrument including a slave box, a transmission and an instrument end assembly,

the slave box provided therein with at least one slave component configured to control movement of the instrument end assembly by means of the transmission; and

the drive box according to any of the preceding paragraphs, the transmission assembly in the drive box configured to be coupled to the slave component to drive the slave component and hence the instrument end assembly to move.

Optionally, the surgical instrument system may further include a sterile adaptor detachably coupled to the slave component and the transmission assembly, the sterile adaptor configured to transmit kinetic power from the drive box to the slave box.

Optionally, the sterile adaptor may include at least one intermediate rotary disc, wherein the output base is provided at the fourth end with a master connector and the intermediate rotary disc is provided with a slave interface on a surface facing the drive box, wherein one of the master connector and the slave interface is an insertion recess and the other is an insertion block, and wherein inserting the insertion block into the insertion recess results in connection of the output base with the intermediate rotary disc.

Optionally, at least two of the master connectors may be provided so as to be scattered in a non-centrosymmetric manner at the fourth end of the output base, wherein the same number of slave connectors as the number of master connectors are provided in correspondence with the respective master connectors.

Optionally, each of the master connector and the slave interface may define a draft angle.

Optionally, each of the master connector and the slave interface may have a sector-shaped cross section.

In pursuit of the above aim, the present invention also provides a surgical robot system including a side cart including a surgical manipulator arm and the surgical instrument system according to any of the preceding paragraphs, wherein the drive box in the surgical instrument system is provided on the surgical manipulator arm.

The transmission assembly, the drive box, the surgical instrument system and the robot system have the following advantages over the prior art:

First, the transmission assembly includes the input base, the guide transmission and the output base. The guide transmission is disposed on the outer circumferential surface of the input base and configured to transmit a torque to the output base. In this way, the transmission location of the transmission assembly (i.e., the location where the guide transmission cooperatively interacts with the output base) is spaced away from the axis of rotation of the base, resulting in increased transmission accuracy and an improved loading condition of the transmission location.

Second, inserting the second end of the input base into the cavity defined at the third end of the output base enables the transmission assembly to have a reduced axial dimension, which can facilitate miniaturization of the transmission assembly.

Third, the drive mechanism is coupled to the transmission assembly by means of the mounting hole provided at the first end of the input base and is fastened using the locking element. In this way, detachable connection of the drive mechanism with the input base is achieved, which is advantageous in allowing easy assembly and disassembly.

## BRIEF DESCRIPTION OF THE DRAWINGS

Fig. 1 is a schematic illustration of a surgical robot system in operation, according to an embodiment of the present invention;

Fig. 2a schematically illustrates how a slave box, a sterile adaptor and a drive box in the surgical robot system are connected, according to an embodiment of the present invention;

Fig. 2b schematically illustrates the slave box, the sterile adaptor and the drive box in the surgical robot system of Fig. 2a, which are disassembled from one another, showing the drive box not being provided with a transmission assembly in one of its output holes;

Fig. 3a is a schematic diagram showing the structure of the transmission assembly, according to an embodiment of the present invention;

Fig. 3b is a schematic exploded view of the transmission assembly of Fig. 3a;

Fig. 3c is a schematic diagram showing the structure of an input base in the transmission assembly of Fig. 3a, as viewed from one direction;

Fig. 3d is a schematic diagram showing the structure of the input base of Fig. 3c, as viewed from another direction;

Fig. 3e is a schematic diagram showing the structure of an output base in the transmission assembly of Fig. 3a, as viewed from one direction;

Fig. 3f is a schematic diagram showing the structure of the output base of Fig. 3e, as viewed from another direction;

Fig. 3g is an axial cutaway view of the output base of Fig. 3f;

Fig. 3h is a schematic three-dimensional view of a torque transmission surface, according to an embodiment of the present invention;

Fig. 3i schematically illustrates a torque transmission zone, according to an embodiment of the present invention;

Fig. 3j is a schematic illustration of the torque transmission surface, according to an embodiment of the present invention;

Fig. 4 is a schematic diagram showing the structure of the transmission assembly, according to an embodiment of the present invention;

Fig. 5 schematically illustrates the transmission assembly and a drive mechanism, according to an embodiment of the present invention;

Fig. 6 schematically illustrates the transmission assembly and the drive mechanism, according to an embodiment of the present invention;

Fig. 7 schematically illustrates how the input and output bases in the transmission assembly are positioned relative to each other during power transmission thereof, according to an embodiment of the present invention;

Fig. 8a is a cutaway view showing the input base and output base being spaced at a maximum distance during power transmission of the transmission assembly of Fig. 3a;

Fig. 8b is a cutaway view showing the input base and output base being spaced at a minimum distance during power transmission of the transmission assembly of Fig. 8a;

Fig. 9a is a schematic diagram showing the structure of the transmission assembly, according to an embodiment of the present invention, as viewed from one direction;

Fig. 9b is a schematic diagram showing the structure of the transmission assembly of Fig. 9a, as viewed from another direction;

Fig. 9c is a schematic exploded view of the transmission assembly of Fig. 9a;

Fig. 10a is a cutaway view showing the input base and output base being spaced at the maximum distance during power transmission of the transmission assembly of Fig. 9a;

Fig. 10b is a cutaway view showing the input base and output base being spaced at the minimum distance during power transmission of the transmission assembly of Fig. 9a;

Fig. 11a schematically illustrates an axial projection of the transmission assembly, according to an embodiment of the present invention;

Fig. 11b schematically illustrates an axial projection of the transmission assembly, according to an embodiment of the present invention, with an angle $\gamma$ being minimized;

Fig. 11c schematically illustrates an axial projection of the transmission assembly, according to an embodiment of the present invention, with the angle $\gamma$ being maximized;

Fig. 12a is a schematic diagram showing the structure of the transmission assembly, according to an embodiment of the present invention;

Fig. 12b is a schematic exploded view of the transmission assembly of Fig. 12a;

Fig. 12c is a schematic illustration of the transmission assembly of Fig. 12b, as viewed from another direction;

Fig. 13a is a top view of an intermediate rotary disc in the drive box, according to an embodiment of the present invention;

Fig. 13b is a bottom view of the intermediate rotary disc in the drive box, according to an embodiment of the present invention;

Fig. 14 schematically illustrates a processing of engaging a master connector in the drive box with a slave connector in the sterile adaptor, according to an embodiment of the present invention; and

Figs. 15a and 15b schematically illustrates the master connector in the drive box and the slave connector in engagement with each other, according to an embodiment of the present invention.

In these figures:

10 - surgeon's console; 20 - surgical cart; 30 - side cart;

100 - drive box;

1000 - transmission assembly;

1100 - input base;

1110 - receptacle; 1120 - mounting hole; 1130 - locking hole; 1140 - locating groove;

1200 - guide transmission;

1300 - output base;

1310 - mating internal groove; 1320 - mating external groove; 1330 - master connector; 1340 - through mounting hole; 1350 - recess; 1360 - torque transmission surface;

1400 - axial energy storage member;

1511 - first stopper; 1512 - first limiting stopper; 1513 - first screw hole; 1514 - third stopper; 1515 - third limiting stopper;

1521 - second stopper;

1600 - cover plate;

1700 - locking element;

2000 - drive mechanism;

3000 - housing;

3100 - output hole;

200 - slave box;

300 - surgical instrument;

400 - sterile adaptor;

410 - plate body; 420 - intermediate rotary disc; and 430 - slave connector.

## DETAILED DESCRIPTION

Objects, advantages and features of the present invention will become more apparent from the following more detailed description of embodiments of the transmission assembly, drive box, surgical manipulator arm and surgical robot provided in the invention, when taken in conjunction with the drawings. Note that the figures are provided in a very simplified form not necessarily drawn to exact scale for the only purpose of helping to explain the disclosed embodiments in a more convenient and clearer way.

As used herein, the singular forms "a", "an" and "the" include plural referents, and the phrase "a plurality of" is used in the sense including "two or more", unless the context clearly dictates otherwise. As used herein, the term "or" is employed in the sense including "and/or", unless the context clearly dictates otherwise. Further, the terms "installation", "connection" and "coupling" should be interpreted in a broad sense. For example, a connection may be a permanent, detachable or integral connection, or a mechanical or electrical connection, or a direct or indirect connection with one or more intervening elements, or an internal communication or interaction between two individual components. Those of ordinary skill in the art can understand the specific meanings of the above-mentioned terms herein according to the specific circumstances. Throughout the accompanying drawings, like reference numerals refer to like elements.

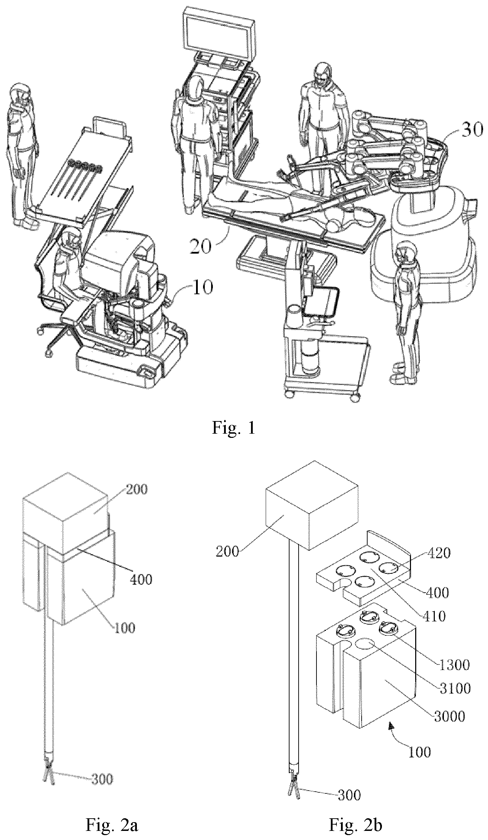

Fig. 1 is a schematic illustration of a surgical robot system in operation according to an embodiment of the present invention. As shown in Fig. 1, the surgical robot system includes a controller end and an effector end. The controller end includes a surgeon's console 10 provided with a master manipulator and the effector end includes a surgical cart 20, a side cart 30 and other equipment. A patient is operated on while lying on the surgical cart 20. A surgical manipulator arm (not labeled) for mounting thereon of a surgical instrument is disposed on the side cart 30. The surgical manipulator arm and the surgical instrument system are mapped in advance to, and thus slaved by, the master manipulator. The surgical manipulator arm and the surgical instrument system move following movement of the master manipulator so that the surgical instrument is manipulated to take actions in various directions, thus accomplishing surgical manipulation.

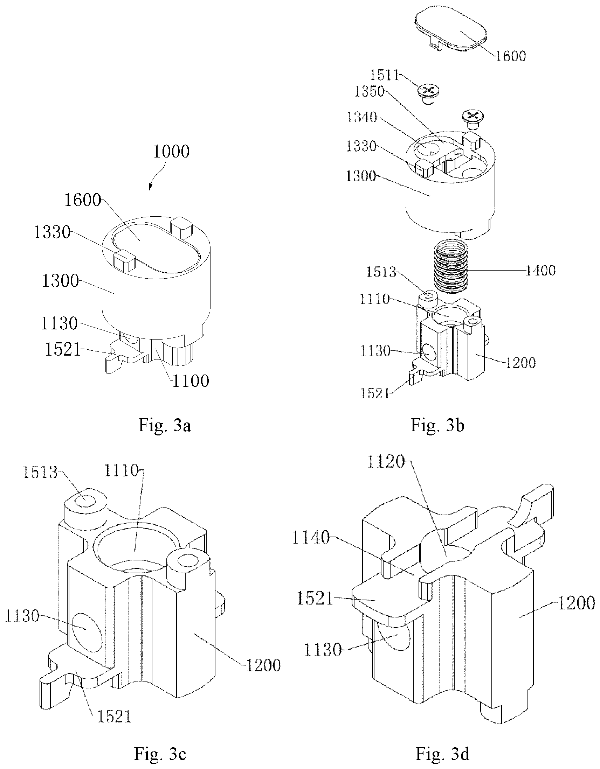

Specifically, as shown in Figs. 2a and 2b, the surgical instrument system includes a drive box 100 and a surgical instrument. The surgical instrument includes a proximal slave box 200, a cable transmission member and a distal instrument end assembly. The instrument end assembly includes at least one joint, and the cable transmission member is adapted to transmit kinetic power from the slave box 200 to the joint in the instrument end assembly, thereby driving this joint to move. Preferably, the surgical instrument further includes a hollow instrument shaft (not labeled) between the slave box 200 and the instrument end assembly. The cable transmission member is passed through the hollow instrument shaft from the slave box 200 to the instrument end assembly. Additionally, the instrument end assembly includes an end effector, the end effector includes an effector assembly. Preferably, the effector assembly is capable of opening and closing movements by virtue of an opening/closing joint. It is to be appreciated that examples of the end effector include, but are not limited to, forceps, scissors, graspers, needle holders, cutting blades, staplers, etc. Preferably, the instrument end assembly further includes a pitch joint adapted to drive the end effector to make pitch movements. More preferably, an axis of the pitch joint is perpendicular to an axis of the opening/closing joint. Additionally, the slave box 200 is provided therein with a number of slave components (not shown), each connected to the surgical instrument 300 by the transmission in order to control movement of the surgical instrument 300. The drive box 100 is disposed on the surgical manipulator arm. The drive box 100 is provided therein with a transmission assembly (as shown in Fig. 3a) and a drive mechanism. The transmission assembly is driven by the drive mechanism (not shown in Fig. 3a) to provide a driving force to the slave components in the slave box 200. In addition, the surgical instrument system further includes a sterile adaptor 400. The entire surgical robot system must be sterilized before surgery. However, since the surgical manipulator arm and the drive box 100 in the surgical instrument system both contain active devices, they are not suitable to be sterilized using conventional methods. For this reason, a sterile bag is disposed between the surgical instrument and the drive box 100 and even the surgical manipulator arm to provide them with a sterilization treatment. The sterile adaptor 400 is arranged on the sterile bag as a power interface between the non-sterile and sterile environments for transmitting kinetic power from the drive box 100 to the slave box 200 in the surgical instrument.

As shown in Figs. 3a and 3b, it is a first object of embodiments of the present invention to provide a transmission assembly 1000 suitable for use in a surgical robot system. Specifically, the transmission assembly 1000 includes an input base 1100, a guide transmission 1200, an output base 1300, an axial energy storage member 1400 and an axial limiting assembly. The input base 1100 has a first end (not labeled), an opposing second end (not labeled) and an outer circumferential surface (not labeled) adapted to connect the first and second ends. The first end is adapted to connect a drive mechanism. The guide transmission 1200 is disposed on the outer circumferential surface of the input base 1100. The output base 1300 has a third end (not labeled) and an opposing fourth end (not labeled). The third end is closer to the first end than the fourth end. The axial energy storage member 1400 is adapted to store energy when the input base 1100 and the output base 1300 are caused by an external force to approach each other. When the external force is removed, the energy will be released, causing the input base 1100 and the output base 1300 to move away from each other. The axial limiting assembly is adapted to limit a maximum displacement of the input base 1100 and the output base 1300 from each other. The output base 1300 is configured to match the shape of the guide transmission 1200, and the guide transmission 1200 is adapted to drive, while the input base 1100 is rotating under the drive of the drive mechanism, the output base 1300 to rotate in sync with the input base 1100.

Figs. 3a to 3g are schematic diagrams illustrating in detail the structure of a first embodiment of the transmission assembly 1000. In this embodiment, the third end of the output base 1300 is an open end, and the output base 1300 defines a cavity (not labeled) in which the second end of the input base 1100 is received.

With continued reference to Figs. 3a to 3g, the guide transmission 1200 is defined by an external extension of the outer circumferential surface of the input base 1100. Correspondingly, an inner side wall of the output base 1300 defines a mating internal groove 1310 for receiving the guide transmission 1200, the mating internal groove 1310 is adapted in shape and size to the guide transmission 1200. For example, if a cross-sectional shape of the guide transmission 1200 is rectangular, then a cross-sectional shape of the mating internal groove 1310 may be like "]". When the input base 1100 is rotated by a driving force about its axis, the guide transmission 1200 will transfer a torque to the output base 1300 through the mating internal groove 1310, causing the output base 1300 to move in sync with the input base 1100. Preferably, a maximum width of the guide transmission 1200 is defined along the axis of the input base 1100, in order to allow the guide transmission 1200 to have a larger force transmission surface. In addition, the present embodiment is not limited to any particular number of the guide transmissions 1200, and there may be either one or more guide transmissions 1200. In the latter case, the multiple guide transmissions 1200 may be spaced apart on the outer circumferential surface of the input base 1100. Further, in order to avoid accidental occurrence of circumferential movement of the output base 1300 during its movement along the direction of extension of the guide transmission 1200, the direction of extension of the guide transmission 1200 shall be parallel to the axis of the input base 1100.

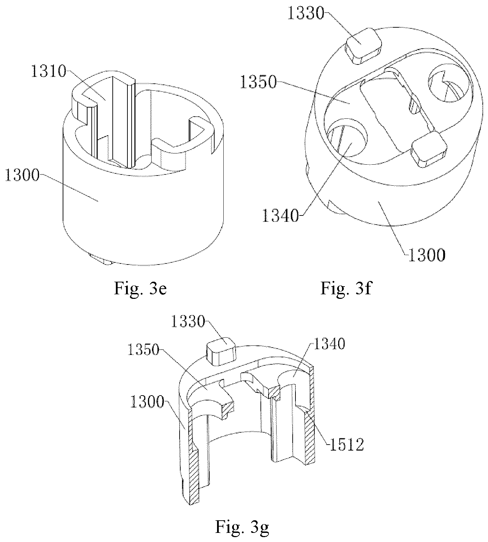

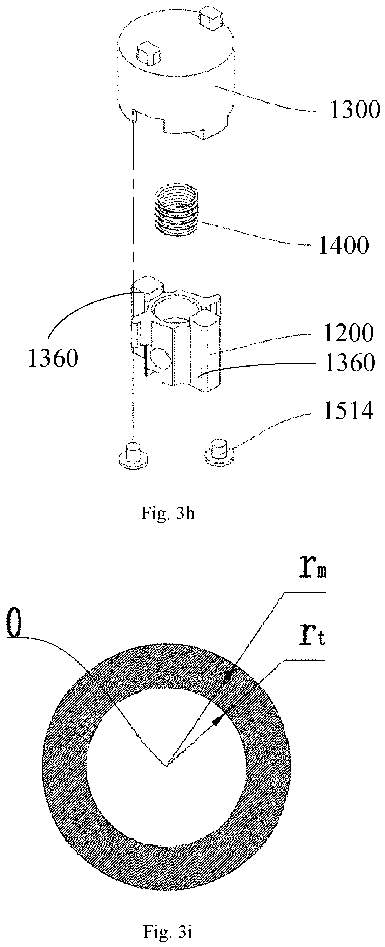

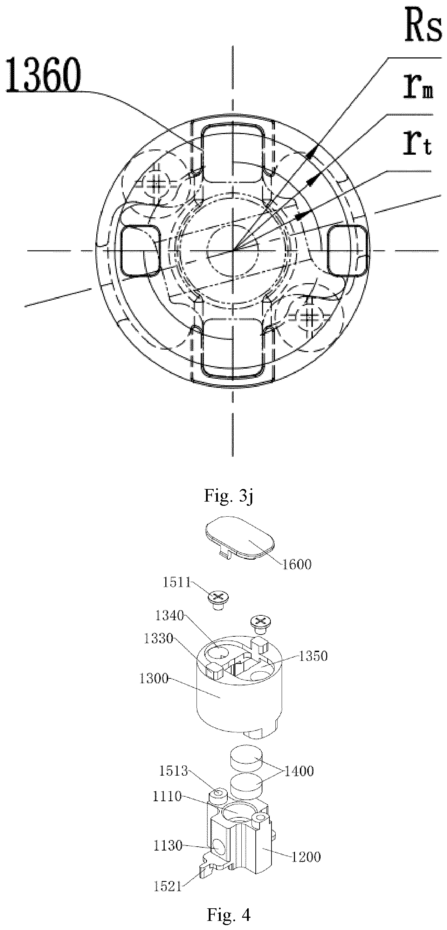

Reference is additionally made to Figs. 3h to 3j. As shown in Fig. 3h, the mating internal groove 1310 (see Fig. 3e) cooperates with the guide transmission 1200 to define a torque transmission surface 1360. As shown in Figs. 3i and 3j, the torque transmission surface 1360 is spaced from a center O of the output base 1300 proximally by a distance $r_t$ and distally by a distance $r_m$. As shown in Fig. 3i, the torque transmission surface 1360 and any torque thereon are situated between a first imaginary circle centered at the center O of the output base 1300 and having a radius of $r_t$ and a second imaginary circle centered at the center O of the output base 1300 and having a radius of $r_m$. As shown in Fig. 3j, the proximal distance $r_t$ from the torque transmission surface 1360 to the center O of the output base 1300 satisfies the relationship $0.3R_S \leq r_t < R_S$, and the distal distance $r_m$ from the torque transmission surface 1360 to the center O of the output base 1300 satisfies the relationship $r_t < r_m \leq R_S$. Moreover, $(r_m - r_t) \in [0.03Rs, 0.7Rs]$, where Rs represents a distance between the center and the most distant boundary of the output base 1300 from the center. As shown, Rs is a radius of a third imaginary circle that is centered at the center O of the output base 1300, and the most distant boundary of the output base 1300 from the center is situated on the third imaginary circle. In this way, at a given assembly clearance, fewer transmission errors will be made. Moreover, under a given torque transmission condition, the components will be less stressed and thus improved in terms of service life and reliability. More preferably, the proximal distance $r_t$ from the torque transmission surface 1360 to the center O of the output base 1300 lies between $0.7 R_S$ and $R_S$, in order to result in additional reductions in transmission error and stress.

In embodiments of the present application, the transmission assembly 1000 further includes a rolling member (not shown) disposed between the guide transmission 1200 and the mating internal groove 1310 to accomplish torque transmission between the guide transmission 1310 and the mating internal groove 1310. The rolling member includes rolling elements and a guide channel, which are separately provided on the guide transmission 1200 and the mating internal groove 1310. Specifically, the rolling elements may be provided on the guide transmission 1200, and the guide channel on the mating internal groove 1310. Alternatively, the rolling elements may be provided on the mating internal groove 1310, and the guide channel on the guide transmission 1200. The rolling elements include at least one roller and a roller cage in which each roller is arranged so as to be rollable in the guide channel. The roller cage is adapted to keep each roller at a constant position relative to the roller cage, and the guide channel is arranged along an axis of the transmission assembly 1000. Here, the rolling elements include a plurality of rollers, and the roller cage keeps these rollers at constant positions relative to one another. Additionally, the rollers may be spherical or cylindrical rollers. For example, the rolling member includes a plurality of cylindrical rollers, which are arranged in a line in the roller cage. Alternatively, the cylindrical rollers are arranged in the roller cage in a staggered manner to form a structure like that of a ball bearing or of a roller bearing.

As shown in Fig. 3b, the axial energy storage member 1400 has the characteristic of storing energy when stressed and releasing the stored energy when the stress is removed. The axial energy storage member 1400 is arranged between the input base 1100 and the output base 1300. As such, when the transmission assembly 1000 is subject to an axial force when it is brought into connection with an external component, the output base 1300 will approach the input base 1100 along the direction of the axis of the input base 1100, and the axial energy storage member 1400 will store energy. When the axial force is removed after the connection of the transmission assembly 1000 with the external component has been accomplished, the axial energy storage member 1400 will release the energy, thus causing relative movement of the input base 1100 and the output base 1300 away from each other.

With continued reference to Fig. 3b, the axial energy storage member 1400 may be an elastic element. The elastic element includes, but is not limited to, a spring. One end of the elastic element is brought into abutment against the second end of the input base 1100, and the other end is brought into abutment against an inner wall of the output base 1300 at the fourth end thereof. As shown in Fig. 3c, the input base 1100 defines a receptacle 1110 at the second end, the receptacle 1110 has a stepped surface. The axial energy storage member 1400 is received in the receptacle 1110 so as to abut against the stepped surface. The receptacle 1110 is preferably coaxial with the input base 1100 in order to achieve a stress balance of the input base 1100 and the output base 1300. In this embodiment, only one elastic element is provided so as to be received in the receptacle 1110 at one end and abut against the inner wall of the output base 1300 at the fourth end thereof outside the receptacle 1110 at the other end.

In another implementation, as shown in Fig. 4, the axial energy storage member 1400 may consist of a pair of mutually repulsive magnetic elements, one of which is provided in the receptacle 1110 of the input base 1100, and the other on an inner wall of the output base 1300 at the third end.

In still other variants (not shown), a plurality of receptacles may be provided at the second end of the input base 1100 and scattered evenly around the axis of the input base 1100. For each of the receptacles, a respective one of the axial energy storage member 1400 may be provided. Additionally, the output base 1300 may have a corresponding arrangement. This design can achieve a similar effect.

With continued reference to Fig. 3b, in conjunction with Figs. 3c, 7 and 8a, the axial limiting assembly is provided to limit a maximum displacement for axial movement of the output base 1300 along the axis of the input base 1100.

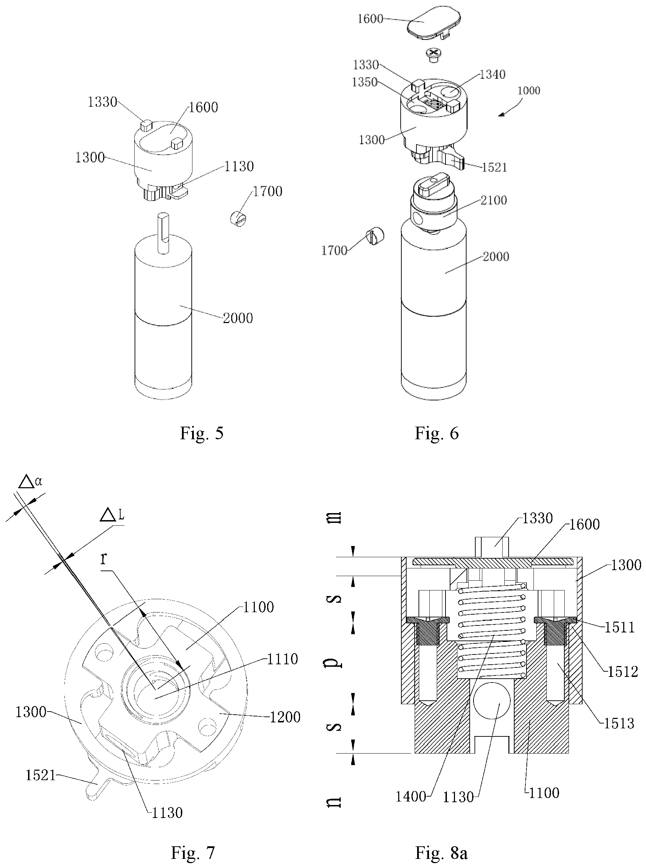

Specifically, the axial limiting assembly includes a first axial limiting sub-assembly, the first axial limiting sub-assembly includes a first stopper 1511 and a first limiting stopper 1512 (as shown in Fig. 8a). The first axial limiting sub-assembly can be adapted to define a first limit position for movement of the output base 1300 away from the input base 1100, thereby determining a maximum length of the transmission assembly 1000. For example, the first stopper 1511 is provided on an end face of the input base 1100 at the second end, and the first limiting stopper 1512 on the inner side wall of the output base 1300. Upon axial movement of the output base 1300 away from the input base 1100 resulting in the first stopper 1511 and the first limiting stopper 1512 coming into interaction with each other, the output base 1300 is stopped from further axially moving away from the input base 1100, i.e., the output base 1300 reaches the first limit position. The interaction of the first stopper 1511 with the first limiting stopper 1512 can also prevent dislodgement of the output base 1300 from the second end of the input base 1100.

As shown in Figs. 3c, 3e, 3f and 3g, in the present embodiment, the first stopper 1511 may be a first screw, and the first limiting stopper 1512 may be a limiting step. The first limiting stopper 1512 is provided on the inner side wall of the output base 1300 so as to radially extend inwardly from the inner side wall. More specifically, a first screw hole 1513 is provided in the end face of the input base 1100 at the second end. The first screw hole 1513 is preferably provided in an end face of the guide transmission 1200. Moreover, the first limiting stopper 1512 is provided on the inner side wall of the output base 1300, at least externally to the first screw hole 1513. In order to facilitate the fabrication, the first limiting stopper 1512 extents around the entire circumference of the inner side wall of the output base 1300, or around one half, one third or one quarter thereof. Preferably, the output base 1300 is provided at the fourth end with an axially extending through mounting hole 1340 that is coaxial with the first screw hole 1513. During assembly, after the output base 1300 is disposed over the input base 1100, the first screw is inserted through the through mounting hole 1340 in the output base 1300 and threadedly connected to the first screw hole 1513 in the input base 1100. This arrangement allows easy assembly of the input base 1100, the output base 1300, the axial energy storage member 1400 and the first axial limiting sub-assembly.

Optionally, as shown in Fig. 3f, a recess 1350 is formed in an external wall at the fourth end of the output base 1300, the through mounting hole 1340 may be provided in the recess 1350. Referring to Fig. 3b, the transmission assembly 1000 further includes a cover plate 1600 for dust prevention. The cover plate 1600 is adapted to fit in the recess 1350 to prevent the ingress of dust and other foreign matter into an internal cavity of the output base 1300. Preferably, an outer surface of the cover plate 1600 makes up a flat surface together with the fourth end of the output base 1300. Further, the cover plate 1600 may be detachably connected to the output base 1300 by snapping, fastening or otherwise.

The axial limiting assembly may further include a second axial limiting sub-assembly (not labeled) capable of defining a second limit position for movement of the output base 1300 toward the input base 1100, thus determining a minimum length of the transmission assembly 1000. The second axial limiting sub-assembly may take one of many forms. For example, the second axial limiting sub-assembly may include a second stopper 1521 and a second limiting stopper. Upon axial movement of the output base 1300 toward the input base 1100 resulting in the second stopper 1521 and the second limiting stopper coming into interaction with each other, the output base 1300 is stopped from further axially approaching the input base 1100, i.e., the output base 1300 reaches the second limit position. Specifically, the second stopper 1521 is disposed on the outer circumferential surface of the input base 1100 proximal to the first end (as shown in Fig. 3b). Preferably, the second stopper 1521 is located between adjacent two of the guide transmissions 1200. The second limiting stopper is provided on an end face of the output base 1300 at the third end. Preferably, the second limiting stopper is integrally formed with the output base 1300. During movement of the output base 1300 toward the input base 1100, i.e., during axial downward movement of the output base 1300, upon the second limiting stopper on the lower end face of the output base 1300 coming into abutment against the second stopper 1521, the output base 1300 reaches the second limit position.

As shown in Fig. 5, according to embodiments of the present invention, the transmission assembly 1000 is adapted to be connected to a drive mechanism 2000 and driven thereby to rotate about the own axis of the drive mechanism 2000. The drive mechanism 2000 may be implemented as a motor. As actually needed, the drive mechanism 2000 may be further equipped with a speed reducing mechanism (not shown). The drive mechanism 2000 has an output shaft and is preferred to be detachably connected to the transmission assembly 1000 in order to permit replacement after damage.

With combined reference to Fig. 3d, the input base 1100 is provided at the first end with a mounting hole 1120 adapted to enable the input base 1100 to be connected to the output shaft of the drive mechanism 2000. The mounting hole 1120 is preferably arranged coaxially with the input base 1100. Additionally, the input base 1100 is further provided with a locking hole 1130 and a locking element 1700. The locking hole 1130 may work with the locking element 1700 to secure the drive mechanism 2000 to the input base 1100. Specifically, the locking hole 1130 may be implemented as a threaded hole, and the locking element 1700 as a threaded connecting element (e.g., a screw). Alternatively, the locking hole 1130 may be implemented as a pin hole, and the locking element 1700 as a biased pin or regular pin shaft.

Fig. 5 shows one method of connecting the transmission assembly 1000 to the drive mechanism 2000 according to the present embodiment. In this example, the locking hole 1130 is brought into communication with the mounting hole 1120. More specifically, the locking hole 1130 is provided at a location of the outer circumferential surface of the input base 1100 that is close to the first end. After the output shaft of the drive mechanism 2000 is inserted into the mounting hole 1120, the locking element 1700 may be inserted through the locking hole 1130 to exert a locking force on the output shaft of the drive mechanism 2000 and thereby connect it to the input base 1100.

It is to be understood that, in the aforementioned input base 1100, the mounting hole 1120 may be either brought into communication with the receptacle 1110 or not. However, it is to be noted that, in the case of the mounting hole 1120 being brought into communication with the receptacle 1110, an abutment shoulder (e.g., defined by an unlabeled stepped surface) shall be provided between them for supporting the axial energy storage member 1400.

In an alternative embodiment, the drive mechanism 2000 has a profiled end portion. The "profiled end portion" refers to an end portion having a non-circular cross section. Correspondingly, the mounting hole 1120 of the transmission assembly 1000 is also profiled. This arrangement allows the transmission assembly 1000 and the drive mechanism 2000 to be maintained circumferentially stationary relative to each other. Additionally, the locking hole 1130 includes a first locking hole section and a second locking hole section. The second locking hole section is provided at one end of the profiled end portion of the drive mechanism 2000, and the first locking hole section is adapted to be brought into communication with the mounting hole 1120 and the receptacle 1110. The locking element 1700 is inserted through the first locking hole section and connected to the second locking hole section (e.g., detachably by snapping, threading, or otherwise), thus connecting the transmission assembly 1000 to the drive mechanism 2000 and making them axially stationary relative to each other.

Fig. 6 shows another method of connecting the transmission assembly 1000 to the drive mechanism 2000 according to the present embodiment. In this method, the transmission assembly 1000 is indirectly coupled to the drive mechanism 2000. Specifically, the connection of the transmission assembly 1000 with the drive mechanism 2000 is accomplished using a coupling. The added coupling may be further provided thereon with an additional supporting component such as a gear transmission, an encoder or a sensor for detecting an output speed, position, torque and other information of the reducer's shaft. At the end of the coupling where it is connected to the drive mechanism 2000, there is provided a mounting hole for receiving the output shaft of the drive mechanism 2000. Similarly, the coupling is further provided with a locking hole and a locking element, which cooperate with each other to fixedly connect the coupling to the drive mechanism 2000. A portion of the coupling at the end where it is connected to the transmission assembly 1000 is profiled. Correspondingly, the mounting hole 1120 in the transmission assembly 1000 is also profiled. This arrangement allows the coupling and the drive mechanism 2000 to be maintained circumferentially stationary relative to each other. Additionally, the second locking hole section is provided at the end of the coupling corresponding to the transmission assembly 1000, and the first locking hole section is brought into communication with the mounting hole 1120 and the receptacle 1110. The second and first locking hole sections of the locking element 1700 work together to connect the input base 1100 and the coupling, keeping the input base 1100 and the coupling axially stationary relative to each other.

Hereinafter, transmission accuracy, a loading condition of a force transmission location, utilization of the axial dimension and other factors of the transmission assembly 1000 of the foregoing embodiments will be analyzed.

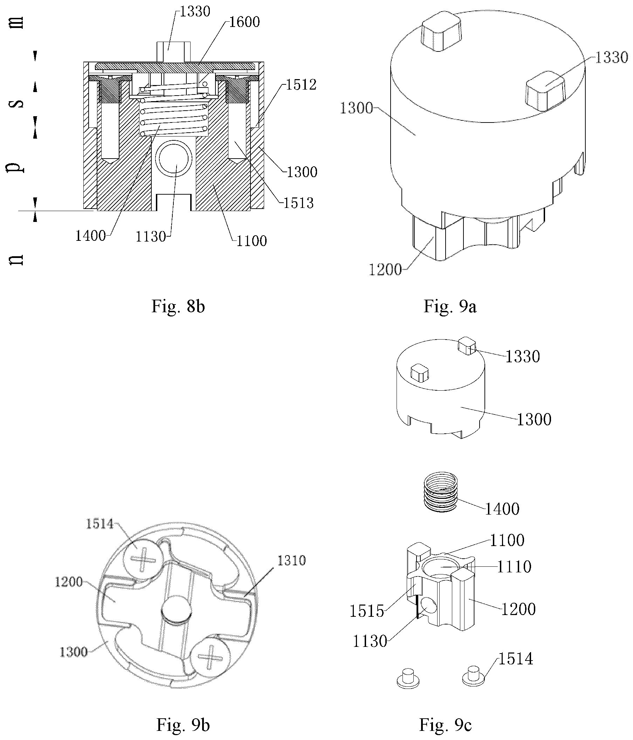

Fig. 7 schematically illustrates how the input base 1100 and the output base 1300 in the transmission assembly 1000 cooperate with each other for transmission. Fig. 8a is a schematic structural view showing the input base 1100 and the output base 1300, which are spaced at the maximum axial distance, i.e., showing the output base 1300 having moved upward to the first limit position. Fig. 8b is a schematic structural view showing the input base 1100 and the output base 1300, which are spaced at the minimum axial distance, i.e., showing the output base 1300 having moved downward to the second limit position.

Angular accuracy for mechanical transmission is given by:

$$\Delta\alpha = \frac{\Delta l}{r} \qquad (\text{I})$$

where $\Delta\alpha$ represents an angular deviation; $\Delta l$ denotes a circumferential clearance between the input of the transmission assembly and the output shaft; and r is a distance from the transmission location to the axis of transmission assembly, i.e., the distance from the guide transmission 1200 to the center of rotation.

According to equation (I), at a given circumferential clearance $\Delta l$ between the input of the transmission assembly 1000 and the output shaft, a greater distance r between the transmission location and the axis of the transmission assembly 1000 can result in higher transmission accuracy. Therefore, in the embodiments of the present invention, arranging the guide transmission 1200 on the outer circumferential surface of the input base 1100 expands the distance (r) from the transmission location to the axis of the transmission assembly 1000, resulting in improved transmission accuracy of the transmission assembly 1000.

The transmission location of the transmission guide 1200 is subject to a force given by:

$$F \approx \frac{0.5M}{r} \qquad (II)$$

where F represents the force acting on the transmission location of the transmission guide; M denotes a transmitted torque; and r is the distance from the transmission location to the axis of transmission assembly, i.e., the distance from the guide transmission 1200 to the center of rotation.

As can be seen from equation (II), for a given torque to be transmitted, a greater distance (r) from the transmission location to the axis of the input base 1100 can reduce the stress on the transmission location. Similarly, in the embodiments of the present invention, arranging the guide transmission 1200 on the outer circumferential surface of the input base 1100 expands the distance (r) from the transmission location to the axis of the transmission assembly 1000, resulting in a reduced load on the guide transmission 1200.

With combined reference to Figs. 4, 8a and 8b, according to the present embodiment, in the transmission assembly 1000, at the maximum axial distance between the input base 1100 and the output base 1300, the axial dimension of the transmission assembly 1000 is given by:

$$L = n + 2s + p + m \qquad (III)$$

where L is the axial dimension of the transmission assembly 1000, n is an intended clearance corresponding to the minimum distance between the input base 1100 and output base 1200, s is a maximum stroke for axial movement of the output base 1200, p is a minimum overlap length between the input base 1100 and the output base, and m is the sum of axial thicknesses of the first stopper 1511 and the cover plate 1600.

Utilization of the axial dimension of the transmission assembly 1000 will be maximized when the maximum stroke s for axial movement of the output base 1200 is equal to the minimum overlap length p between the input base 1100 and the output base. As can be seen from equation (III), when the maximum stroke s for axial movement of the output base 1200 and the minimum overlap length p between the input base 1100 and the output base are both fixed, the axial dimension of the transmission assembly 1000 is dependent only on the intended clearance corresponding to the minimum distance between the input base 1100 and output base 1200 and the sum m of the axial thickness of the leading portion 1512 of the first axial limiting sub-assembly and the axial thickness of the cover plate 1600. That is, the maximum utilization of the axial dimension is determined by n and m. In practical fabrication processes, n lies in (0 mm, 2 mm] and m can be controlled within [0.2 mm, 4 mm]. Therefore, the number of n and m can be completely neglected. That is, according to the embodiments of the present invention, very high utilization of the axial dimension of the transmission assembly 1000 is enabled, greatly facilitating miniaturization of the transmission assembly 1000.

Figs. 9a to 9c are schematic diagrams showing the structure of a second embodiment of the present invention. In the present embodiment, the input base 1100, the guide transmission 1200 and the axial energy storage member 1400 are configured substantially the same as in the first embodiment. For the sake of brevity, only differences from the first embodiment are set forth below.

In the present embodiment, the output base 1300 is closed at the fourth end. Accordingly, both the first and second axial limiting sub-assemblies are re-configured. In this embodiment, the first axial limiting sub-assembly includes a third stopper 1514 and a third limiting stopper 1515, which are adapted to define the first limit position for movement of the output base 1300 away from the input base 1100. The third stopper 1514 is provided on the end face of the output base 1300 at the third end, and the third limiting stopper 1515 is provided on the outer circumferential surface of the input base 1100 at the second end. Correspondingly, in the internal cavity of the output base 1300, there is provided a receptacle channel matching the shape of the third limiting stopper 1515, facilitating axial movement of the third limiting stopper 1515. With this arrangement, when the third stopper 1514 comes interaction with the third limiting stopper 1515, the output base 1300 will reach the first limit position where it is axially spaced apart from the input base 1100 at the maximum distance.

In this embodiment, the third stopper 1514 may be implemented as a second screw, the third limiting stopper 1515 is disposed on the outer circumferential surface of the input base 1100 at the second end so as to radially extend outwardly from the input base 1100. The limiting function of the first axial limiting sub-assembly can be accomplished by providing a second screw hole in the end face of the output base 1300 at the third end so as to be aligned with the third limiting stopper 1515 and by disposing the second screw in the second screw hole. At the distance between the output base 1300 and the input base 1100 that corresponds to the first limit position, a head of the second screw is directed toward a surface of the output base 1300 and abuts against the third limiting stopper 1515. The first axial limiting sub-assembly made up of the second screw and the third limiting stopper 1515 can be conveniently assembled with the input base 1100 and the output base 1300.

In this embodiment, the second axial limiting sub-assembly is also adapted to define the second limit position for movement of the output base 1300 toward the input base 1100. The second axial limiting sub-assembly includes a fourth stopper and a fourth limiting stopper. Upon axial movement of the output base 1300 toward the input base 1100 resulting in the fourth stopper and the fourth limiting stopper coming into interaction with each other, the output base 1300 is stopped from further axially approaching the input base 1100, i.e., the output base 1300 reaches the second limit position. Specifically, the fourth stopper is provided on the end face of the input base 1100 at the second end, and the fourth limiting stopper on the inner wall of the output base 1300 at the fourth end. Preferably, the fourth stopper is provided on an end face of the guide transmission 1200 facing the output base 1300. The fourth stopper and the third limiting stopper 1515 are arranged with a height difference being formed therebetween, which allows the entire transmission assembly 1000 to have a reduced weight, entailing a light-weight design of the product. Preferably, the fourth limiting stopper is integrally formed with the inner wall of the output base 1300 at the fourth end, and the fourth stopper is integrally formed with the guide transmission 1200. Upon movement of the output base 1300 toward the input base 1100, i.e., axial downward movement thereof, resulting in the fourth limiting stopper on the inner wall of the output base 1300 at the fourth end coming into abutment against the fourth stopper 1521 of the guide transmission 1200, the output base 1300 reaches the second limit position.

The transmission accuracy and loading condition of the transmission location of the transmission assembly 1000 in the present embodiment are substantially the same as those of the transmission assembly in the first embodiment.

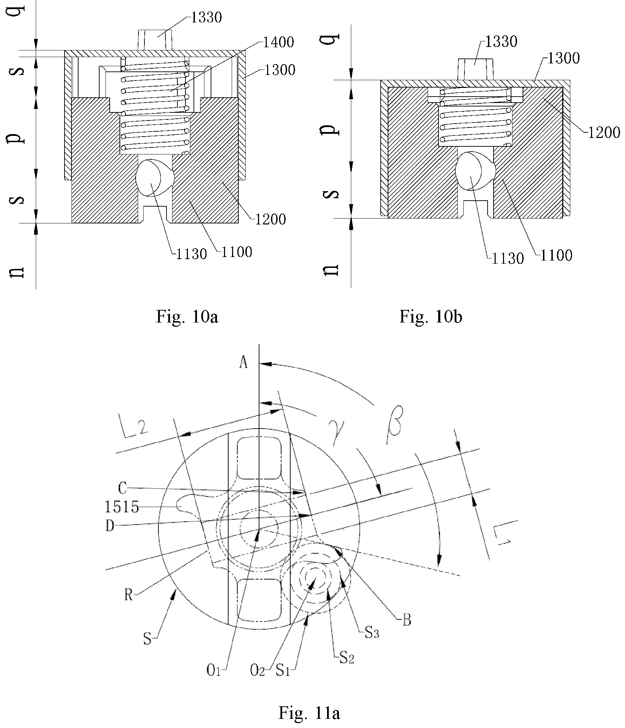

As to utilization of the axial dimension of the transmission assembly 1000 in this embodiment, reference can be made to Figs. 10a and 10b. Fig. 10a is a schematic diagram showing the output base 1300 and the input base 1100, which are spaced at the maximum axial distance, i.e., showing the output base 1300 having moved upward to the first limit position. Fig. 10b is a schematic diagram showing the output base 1300 and the input base 1100, which are spaced at the minimum axial distance, i.e., showing the output base 1300 having moved downward to the second limit position. The maximum axial dimension of the transmission assembly 1000 is given by:

$$L = n + 2s + p + q \qquad (IV)$$

where L is the axial dimension of the transmission assembly 1000, n is an intended clearance corresponding to the minimum distance between the input base 1100 and output base 1300, s is a maximum stroke for axial movement of the output base 1300, p is a minimum overlap length between the input base 1100 and the output base, and q is a wall thickness of the output base 1300 at the fourth end (including the fourth limiting stopper).

With similarity to the first embodiment, maximum utilization of the axial dimension of the transmission assembly 1000 in the present embodiment is determined by n and q. In the present embodiment, since it is not necessary to provide the through hole 1513 or a recess in the output base 1300 at the fourth end, the output base 1300 is allowed to have a reduced wall thickness q at the fourth end (in practical fabrication processes, q can be controlled within [0.3 mm, 3 mm]), resulting in a reduction in the axial dimension of the transmission assembly 1000 and an increase in the utilization of the axial dimension of the transmission assembly 1000.

The various components in the transmission assembly 1000 of the present embodiment are arranged at relative positions as further detailed below, which enable the transmission assembly 1000 to have a more compact structure and a lighter weight and to be more closely connected to the drive mechanism 2000.

Figs. 11a to 11c are schematic illustrations of axial projections of the transmission assembly 1000. These schematic diagrams show relative circumferential limit positions and size limits of the components in the transmission assembly (e.g., the third limiting stopper 1515, locking hole 1130). For the sake of clarity, some parts are not shown.

The relative position of the third limiting stopper 1515 is determined from the position of the third stopper 1514. The third stopper 1514 includes a limiting end and a connecting end. The output base 1300 is provided with a connecting hole for connection with the connecting end. For example, the third stopper 1514 may be implemented as the aforementioned second screw, with the limiting end as a head, the connecting end as a shank, of the screw, and the connecting hole as the aforementioned second screw hole. Specifically, referring to Figs. 11a to 11c, the transmission assembly 1000 is centered at $O_1$ and is located within a circumscribed circle S centered at $O_1$ and having a diameter of $d_1$. A total width of the mating internal groove 1310 and its wall thickness is w, and the mating internal groove 1310 defines a line of symmetry serving as a basis for determining the relative positions of the various components. The third limiting stopper 1515 starts at point B on the outer circumferential surface of the input base 1100 at the second end, and the second screw hole is centered at point $O_2$. In Figs. 11a to 11c, there are also depicted a first radial line $O_1A$, a second radial line OiB and a fifth radial line $O_1O_2$. The first radial line OiA is just the aforesaid line of symmetry, and the second radial line OiB is a straight line passing through the center $O_1$ of the transmission assembly 1000 and the start point B of the third limiting stopper 1515. The fifth radial line $O_1O_2$ is a straight line passing through the center $O_1$ of the transmission assembly 1000 and the center $O_2$. A first imaginary circle $S_1$, a second imaginary circle $S_2$ and a third imaginary circle $S_3$ are all centered at $O_2$. The first imaginary circle $S_1$ is tangent to the second radial line $O_1B$, and the second imaginary circle $S_2$ has a diameter $d_4$ that is equal to the sum of a diameter of the second screw hole and a preset safe thickness for the threaded wall. The third imaginary circle $S_3$ is an inscribed circle tangent to the outer wall S of the mating internal groove 1310 and having a diameter of $d_2$. That is, it corresponds to the limiting end of the third stopper 1514, e.g., the head of the second screw. Generally speaking, the diameter $d_3$ of the first imaginary circle $S_1$ is 1.1-1.6 times the diameter $d_2$ of the third imaginary circle $S_3$, and the diameter $d_4$ of the second imaginary circle $S_2$ is 0.6-0.8 times the diameter $d_2$ of the third imaginary circle $S_3$.

The start position of the third limiting stopper 1515 can be obtained according to:

$$\sin\theta_1 = \frac{d_3}{d_1 - d_2}$$

$$\sin\theta_2 = \frac{d_2 + w}{d_1 - d_2}$$

Thus, we can obtain:

$$\beta = \pi - \arcsin\frac{d_3}{d_1 - d_2} - \arcsin\frac{d_2 + w}{d_1 - d_2}$$

Since, $d_3$ is 1.1-1.6 times $d_2$,

$$\beta \in [\pi - \arcsin\frac{1.6d_2}{d_1 - d_2} - \arcsin\frac{d_2 + w}{d_1 - d_2}, \pi - \arcsin\frac{1.1d_2}{d_1 - d_2} - \arcsin\frac{d_2 + w}{d_1 - d_2}]$$

where $\theta_1$ is an angle required for the second radial line $O_1B$ to rotate clockwise to the fifth radial line $O_2O_2$, $\theta_2$ is an angle required for the fifth radial line $O_1O_2$ to rotate clockwise to the first radial line $O_1A$, and $\beta$ is an angle required for the first radial line OiA to rotate clockwise to the second radial line OiB. In this way, the start position of the third limiting stopper 1515 can be obtained.

In addition, in the present embodiment, the third limiting stopper 1515 is required to avoid interfering with the second imaginary circle S2. That is, a distance from any point on an outer circumferential contour of the third limiting stopper 1515 to the center $O_2$ of the second imaginary circle $S_2$ is required to be greater than $0.5*d_4$. Since $d_4=0.6-0.8d_2$, the distance from any point on the third limiting stopper 1515 to the center $O_2$ is required to be greater than $0.3*d_2$.

Further, a relative position relationship of the locking hole 1130 is determined from the position of the third stopper 1514. An imaginary rectangle R that is centered at the center $O_1$ of the transmission assembly 1000 and representative of the locking hole 1130 is created. Short sides of the imaginary rectangle represent a diameter of the locking hole 1130, and long sides of the imaginary rectangle represent a length of the locking hole 1130. In order to ensure sufficient connection strength between the transmission assembly and the drive mechanism, it is necessary to preset a length $L_1$ of the short sides of the imaginary rectangle and a length $L_2$ of the long sides of the imaginary rectangle. Point C represents one corner on, point E represents the other corner on, and point D represents a middle point of, the right short side of the imaginary rectangle R.

In Figs. 11a to 11c, a third radial line $O_1C$ and a fourth radial line $O_1D$ are also depicted. The third radial line OiC is a straight line passing through the center $O_1$ of the transmission assembly 1000 and the corner C of the imaginary rectangle R, and the fourth radial line OiD is a straight line passing through the center $O_1$ of the transmission assembly 1000 and the middle point D of the short side of the imaginary rectangle R. Further, the relative position of the locking hole 1130 can be determined in the manner as detailed below.

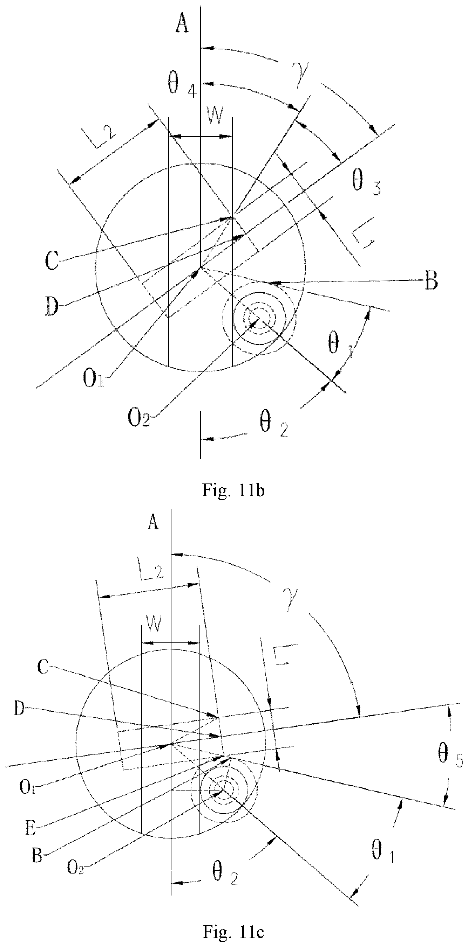

When the corner C of the imaginary rectangle R is just located on the outer wall of the mating internal groove 1310, the angle $\gamma$ is minimized (as shown in Fig. 11b). Accordingly, we can obtain:

$$\tan\theta_3 = \frac{L_1}{L_2} \qquad \sin\theta_4 = \frac{w}{\sqrt{L_1^2 + L_2^2}}$$

Therefore,

$$\gamma_{\min} = \arctan\frac{L_1}{L_2} + \arcsin\frac{w}{\sqrt{L_1^2 + L_2^2}}$$

When the corner E of the imaginary rectangle R is located on the second radial line $O_1B$, the angle $\gamma$ is maximized (as shown in Fig. 11c), and the fourth radial line $O_1D$ is an axis of symmetry for the third radial line $O_1C$ and the second radial line $O_1B$, so we have:

$$\theta_3 = \theta_5 .$$

Therefore,

$$\tan\theta_5 = \tan\theta_3 = \frac{L1}{L2} .$$

Additionally, as $\gamma_{\max} = \beta - \theta_5$,

$$\gamma_{\max} = \pi - \arcsin\frac{d_3}{d_1 - d_2} - \arcsin\frac{d_2 + w}{d_1 - d_2} - \arctan\frac{L_1}{L_2} .$$

Moreover, as $d_3$ is 1.1-1.6 times $d_2$, a value range for the angle $\gamma$ is

$$\gamma_{\max} = \pi - \arcsin\frac{1.1 d_2}{d_1 - d_2} - \arcsin\frac{d_2 + w}{d_1 - d_2} - \arctan\frac{L_1}{L_2} .$$

Finally, the range for the angle $\gamma$ is obtained as:

$$\gamma \in [\arctan\frac{L_1}{L_2} + \arcsin\frac{w}{\sqrt{L_1^2 + L_2^2}} , \pi - \arcsin\frac{1.1 d_2}{d_1 - d_2} - \arcsin\frac{d_2 + w}{d_1 - d_2} - \arctan\frac{L_1}{L_2}] ,$$

where $\gamma$ is the angle required for the first radial line OiA to clockwise rotate to the fourth radial line $O_1D$, $\theta_4$ is an angle required for first radial line OiA to clockwise rotate to the third radial line $O_1C$, $\theta_3$ is an angle required for the third radial line $O_1C$ to clockwise rotate the fourth radial line $O_1D$, and $\theta_5$ is an angle required for the fourth radial line OiD to clockwise rotate to the second radial line OiB.

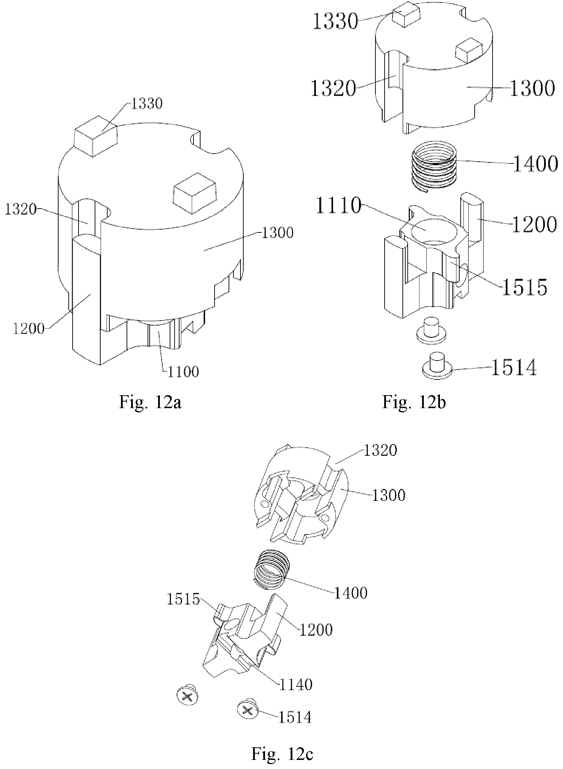

Figs. 12a to 12c are schematic diagrams showing the structure of a third embodiment of the transmission assembly 1000 according to the present invention. This embodiment differs from the second embodiment essentially in the arrangement of the guide transmission 1200.

In particular, in the present embodiment, the guide transmission 1200 is disposed external to the outer circumferential surface of the input base 1100, and the two is connected with a cantilever. Correspondingly, the output base 1300 defines in its outer wall a mating external groove 1320 that matches the guide transmission 1200. In this example, a transmission location of the guide transmission 1200 is further distant from the axis of the input base 1100, resulting in even higher transmission accuracy.

Additional reference can be made to the above description of the mating internal groove 1310 and its torque transmission surface. In this embodiment, the mating external groove 1320 and the guide transmission 1200 cooperate with each other to provide a torque transmission surface. The torque transmission surface 1360 is spaced from the center O of the output base 1300 proximally by a distance $r_t$ and distally by a distance $r_m$. As shown in Fig. 3i, the torque transmission surface 1360 and any torque thereon are situated between a first imaginary circle centered at the center O of the output base 1300 and having a radius of $r_t$ and a second imaginary circle centered at the center O of the output base 1300 and having a radius of $r_m$. As shown in Fig. 3j, the proximal distance $r_t$ from the torque transmission surface 1360 to the center O of the output base 1300 satisfies the relationship $0.3R_S \leq r_t < R_S$, and the distal distance $r_m$ from the torque transmission surface 1360 to the center O of the output base 1300 satisfies the relationship $r_t < r_m \leq R_S$. Moreover, $(r_m - r_t)$ E [0.03Rs, 0.7Rs], where Rs represents a distance between the center and the most distant boundary of the output base 1300 from the center. Likewise, Rs is a radius of a third imaginary circle that is centered at the center O of the output base 1300, and the most distant boundary of the output base 1300 from the center is situated on the third imaginary circle. That is, the third imaginary circle is the largest circumscribed circle of the output base 1300. In this way, at a given assembly clearance, fewer transmission errors will be made. Moreover, under a given torque transmission condition, the components will be less stressed and thus improved in terms of service life and reliability. More preferably, in order to obtain additional reductions in transmission errors and stress, the diameter $r_t$ of the first imaginary circle lies between $0.7R_S$ and $R_S$.

Additionally, the transmission assembly 1000 further includes a rolling member (not shown) disposed between the guide transmission 1200 and the mating external groove 1320 to accomplish torque transmission between the guide transmission 1310 and the mating external groove 1320. The rolling member includes rolling elements and a guide channel, which are separately provided on the guide transmission 1200 and the mating external groove 1320. Specifically, the rolling elements may be provided on the guide transmission 1200, and the guide channel on the mating external groove 1320. Alternatively, the rolling elements may be provided on the mating external groove 1320, and the guide channel on the guide transmission 1200. The rolling elements include at least one roller and a roller cage in which each roller is arranged so as to be rollable in the guide channel. The roller cage is adapted to keep each roller at a constant position relative to the roller cage, and the guide channel is arranged along an axis of the transmission assembly 1000. Here, the rolling elements include a plurality of rollers, and the roller cage keeps these rollers at constant positions relative to one another. Additionally, the rollers may be spherical rollers or cylindrical rollers. For example, the rolling member includes a plurality of cylindrical rollers, which are arranged in a line in the roller cage. Alternatively, the cylindrical rollers are arranged in the roller cage in a staggered manner to form a structure like that of a ball bearing or roller bearing.

On the basis of the transmission assembly 1000 according to the above embodiments, embodiments of the present invention provide a drive box 100. Referring to Figs. 2a, 2b, 5 and 6, the drive box 100 includes a housing 3000, at least one transmission assembly 1000 and at least one drive mechanism 2000. The housing 3000 is provided therein with at least one output hole 3100. The transmission assembly 1000 may be a transmission assembly according to any of the foregoing embodiments and is so disposed within the housing 3000 so as to extend out from the output hole 3100. A fourth end of the transmission assembly 1000 is located outside the housing 3000 in order to allow external transmission of a torque. The drive mechanism 2000 is adapted to be coupled to a first end of an input base 1100 of the transmission assembly 1000 in order to drive the input base 1100 to rotate about its own axis.

Kinetic power provided by each drive mechanism 2000 is transmitted by a respective one of the transmission assembly or transmission assemblies 1000 from a respective one of the output hole or output holes 3100 to a respective surgical instrument 300 to control movement of an instrument end assembly of the surgical instrument 300. That is, the numbers of the output hole or output holes 3100, the transmission assembly or transmission assemblies 1000 and the drive mechanism or drive mechanisms 2000 in the drive box 100 depend on the number of degree(s) of freedom of the surgical instrument 300 to be driven. For example, if the surgical instrument 300 has four degrees of freedom, the numbers of the output hole or output holes 3100, the transmission assembly or transmission assemblies 1000 and the drive mechanism or drive mechanisms 2000 are all four.

Additionally, the input base 1100 is provided at the first end with a mounting hole 1120 for enabling the input base 1100 to be coupled to the drive mechanism 2000. The input base 1100 is also provided with a first locking hole section, and the drive mechanism 2000 is provided with a second locking hole section. A locking element 1700 fits in both the first and second locking hole sections to secure the drive mechanism to the input base 1100. Further, when an output end portion of the drive mechanism 2000 is profiled, the mounting hole 1120 of the transmission assembly 1000 shall have a corresponding shape.

On the basis of the above drive box 100, embodiments of the present invention further provide a surgical instrument system. As shown in Figs. 2a and 2b, the surgical instrument system includes a drive box 100 and a surgical instrument. The surgical instrument includes a proximal slave box 200, a transmission and a distal instrument end assembly. The slave box 200 is provided therein with slave components (not shown), which are connected by the transmission to a joint of the instrument end assembly to drive the joint to move. The drive box 100 includes a transmission assembly 1000, which is detachably connected to the slave components to allow kinetic power to be transmitted from the drive box 100 to the surgical instrument.

In particular, input holes (not shown) for the respective slave components are provided in respective locations of a box body of the slave box 200. The slave components are provided with a slave interface (not shown), which may be detachably coupled to the transmission assembly 1000 in the drive box 100 so as to enable a drive mechanism 2000 in the drive box 100 to drive movement of the slave components and thus of the joint of the instrument end assembly.

With continued reference to Figs. 2a and 2b, the surgical instrument system includes a sterile adaptor 400, which is detachably coupled, as a power transmission medium, to the drive box 100 and the slave box 200.

The sterile adaptor 400 includes a plate body 410 provided with several rotatable intermediate rotary discs 420, which are adapted to be detachably coupled to the slave components and the transmission assembly 1000.

With continued reference to Fig. 2b, in conjunction with Figs. 3a, 9a, 12a, 13a, 13b and 14, a master connector 1330 is provided at a fourth end of an output base 1300 in the transmission assembly 1000, and a corresponding slave connector 430 is provided on the side of a respective one of the intermediate rotary discs 420 facing the output base 1300. In an exemplary embodiment, one of the master connector 1330 and the slave connector 430 is an insertion recess, and the other is an insertion block. In one example, the master connector 1330 is the insertion block, and the slave connector 430 is the insertion recess. Before the insertion block and recess are brought into mating with each other, the intermediate rotary disc 420 exerts an axial force on the output base 1300, causing the output base 1300 to overcome resistance from an axial energy storage member 1400 and move toward the input base 1100. Moreover, under the action of a guide transmission 1200, it rotates in sync with the drive mechanism 2000 relative to the intermediate rotary disc 420. Upon the output base 1300 rotating to an angular position where the insertion block is aligned with the insertion recess, the force on the output base 1300 from the intermediate rotary disc 420 disappears. As a result, the output base 1300 moves away from the input base 1100 under the action of the axial energy storage member, inserting the insertion block into the insertion recess, thus enabling power transmission between the transmission assembly 1000 and the intermediate rotary disc 420.

It should be understood that either one or more of the master connectors 1330 may be provided on the transmission assembly 1000. In particular, in the case of a plurality of master connectors 1330 being provided, these master connectors 1330 are preferably arranged not in symmetry with respect to a center of rotation of an end face at a third end of the output base 1300, and slave connectors 430 are arranged in correspondence with the respective master connectors 1330. In this way, it can be ensured that the transmission assembly 1000 engages the intermediate rotary disc 420 in only one direction, thus ensuring correct control of various joints in the instrument end assembly.

In one embodiment, the insertion block serves as the master connector 1330 and defines a draft angle (e.g., 2°), and the slave connector 430 correspondingly defines an identical draft angle. When the master connector 1330 is inserted into the slave connector 430, the two form a wedge fit which connects the transmission assembly 1000 to the intermediate rotary disc 420.

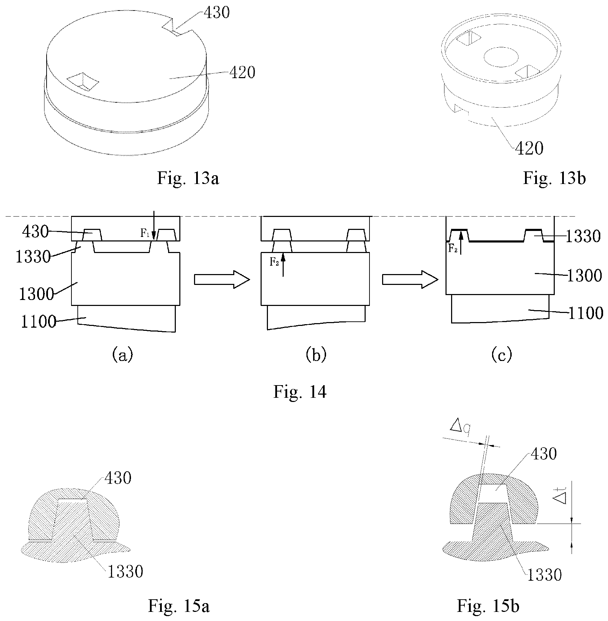

In another embodiment, each of the master connector 1330 and the slave connector 430 is designed with a sector-shaped cross section. Fig. 13a is a schematic bottom view of the intermediate rotary disc 420. As shown in Fig. 13a, the slave connector 430 includes two planar side walls and two curved side walls. Preferably, the planes pass through a center of the slave connector 430, and the curved side walls are arc-shaped side walls. The master connector 1330 is arranged in a similar way. In this way, both the master connector 1330 and the slave connector 430 are more uniformly stressed on their planar surfaces during power transmission. This helps extend the service lives of the master connector 1330 and the slave connector 430.

Additionally, the intermediate rotary disc 420 is provided with a guide wall (not shown) axially extending toward the transmission assembly 1000, and an outer side wall of the output base 1300 proximal to the fourth end is at least partially configured as a surface of revolution. When the output base 1300 is inserted at the fourth end in the guide wall, the surface of revolution will cooperate with the guide wall to achieve concentric positioning of the intermediate rotary disc 420 and the transmission assembly 1000.

Fig. 13b is a schematic top view of the intermediate rotary disc 420. The slave component may be connected to the intermediate rotary disc 420 exactly in the same as the transmission assembly 1000 is connected to the intermediate rotary disc 420. As shown in Fig. 13b, the intermediate rotary disc 420 defines a central interface serving as an insertion recess, while the slave interface is an insertion block that matches the insertion recess.

In the following, a process according to embodiments of the present invention, in which the drive box 100 drives the intermediate rotary discs 420 in the sterile adaptor 400 to rotate and thus transmits power to the slave box 200 in the surgical instrument to cause the surgical instrument to perform an action, will be described with respect to Figs. 2b and 14.

As shown in Fig. 2b, the drive box 100, the sterile adaptor 400 and the surgical instrument 200 are assembled together, with the transmission assemblies 1000, the intermediate rotary discs 420 and the slave components being configured as described previously. Reference is now made to Fig. 14, which schematically illustrates the master connector 1330 and the slave connector 430 in states where they are not matched with each other yet, i.e., the intermediate rotary disc 420 is not being driven by the transmission assembly 1000 to rotate in sync therewith (a), where the master connector 1330 is aligned with the slave connector 430 (b) and where the master connector 1330 mates with the slave connector 430 to couple the intermediate rotary disc 420 to the transmission assembly 1000 (c).

As shown, prior to the mating of the master connector 1330 with the slave connector 430, the master connector 1330 abuts against a surface of the intermediate rotary disc that faces the master connector 1330. In this state, the intermediate rotary disc 420 exerts an axial force $F_1$ directed toward the input base 1100 on the fourth end of the output base 1300, bringing the transmission assembly 1000 in a compressed configuration where the axial energy storage member 1400 stores energy due to the axial force $F_1$. After that, the drive mechanism 2000 drives the transmission assembly 1000 to rotate until the master connector 1330 is aligned with the slave connector 430, as shown in Fig. 14(b). At this point, the axial force $F_1$ disappears, and the axial energy storage member 1400 releases the stored energy and exerts an axial force $F_2$ directed away from the input base 1100 on the output base 1300. The output base is pushed by the axial force $F_2$ to move away from the input base 1100 until the master connector 1330 is inserted into the slave connector 430. In this way, the master connector 1330 mates the slave connector 430, as shown in Fig. 14(c). Thus, the intermediate rotary disc 420 can rotate in sync with the transmission assembly 1000. Subsequently, the sterile adaptor 400 is coupled to the surgical instrument, and the insertion recess of the intermediate rotary disc 420 is further brought into engagement with the slave interface by a similar process. Under an action of the slave interface taken on the intermediate rotary disc 420, both the transmission assembly 1000 and the intermediate rotary disc 420 are compressed. The transmission assembly 1000 and the intermediate rotary disc 420 are then rotated until the central interface of the intermediate rotary disc 420 is aligned with the slave interface. The axial energy storage member 1400 then drives the intermediate rotary disc 420 so that its central interface is coupled to the slave interface. Finally, the drive mechanism 2000 can transmit kinetic power to the surgical instrument through the master components, the sterile adaptor and the slave components to cause movement of a joint in the instrument end assembly.

Referring to Figs. 15a and 15b, in a practical fabrication process, limited by the environment, the used assembly technique and other factors, there will be inevitable deviations of assembly parameters for the master connector 1330 and the slave connector 430. As an example, an axial clearance $\Delta t$ may be present between the master connector 1330 and the slave connector 430. As another example, an angular clearance $\Delta q$ may be present between the master connector 1330 and the slave connector 430 due to the presence of a draft angle. These deviations are detrimental to transmission accuracy. During engagement, the output base 1300 may be pushed by the axial energy storage member 1400 to move axially to insert the master connector 1330 into the slave connector 430 with a tapered fit being formed therebetween, which can compensate for the axial clearance $\Delta t$ and the angular clearance $\Delta q$ and avoid them from exerting an adverse influence on the aforesaid parameters, resulting in an additional increase in transmission accuracy. Preferably, according to embodiments of the present invention, the master connector 1330 and the slave connector 430 may be designed not to be exactly consistent in size. Specifically, the slave connector 430 may have an opening that is sized slightly smaller than a root of the master connector 1330 and a depth that is equal to or slightly greater than a height of the master connector 1330. This design can be ideally combined with the ability of the axial energy storage member 1400 to compensate for fabrication tolerances by axial displacement to result in further increases in transmission accuracy.

Furthermore, embodiments of the present invention also provide a surgical robot system including the above-discussed surgical instrument system. The surgical robot system may include one or more surgical manipulator arms. The drive box 100 in the surgical instrument system is arranged on one of the surgical manipulator arms.

Additionally, although the engagement of the output base 1300 with the input base 1100 has been described in all the foregoing embodiments in the context with the output base 1300 having the aforementioned cavity, in practice, the output base 1300 may not have the cavity. For example, the guide transmission 1200 may extend beyond the second end of the input base 1100, and the output base 1300 may be disposed with the end face at the third end facing the second end of the input base 1100. Additionally, a space for accommodating the axial energy storage member 1400 is defined in both the third and second ends. Therefore, the present invention is not limited to any particular method of engaging the output base 1300 with the input base 1100.

To sum up, embodiments of the present invention provide a transmission assembly having the advantages of satisfactory transmission accuracy, reduced loading of the transmission locations and high utilization of the axial dimension. Embodiments of the present invention also provide a drive box including the transmission assembly and a drive mechanism. This drive box is used in a surgical robot to enable swapping of surgical instruments in various directions and allow the surgical robot to have high operating accuracy and a long service life. Embodiments of the present invention also provide a surgical instrument system and a surgical robot system, each including the drive box.

Although the present invention has been disclosed hereinabove, it is not limited to the above disclosure. Those skilled in the art can make various changes and modifications to the invention without departing from the scope thereof, Accordingly, it is intended that any and all such changes and modifications also fall within the scope of the present invention as defined by the appended claims and equivalents thereof.

**Claims**

1. A transmission assembly (1000) for a surgical robotic system, comprising:

an input base (1100) having a first end and an opposing second end, the first end configured for connection with a drive mechanism (2000);
a guide transmission (1200) disposed on an outer circumferential surface of the input base (1100);
an output base (1300) having a third end and an opposing fourth end, the third end located closer to the first end than the fourth end;
an axial energy storage member (1400) configured to store energy during movement of the input base and the output base (1300) toward each other under the action of an external force and to release, upon removal of the external force, the energy to drive movement of the input base (1100) and the output base (1300) away from each other;
an axial limiting assembly configured to limit a maximum displacement of the input base (1100) and the output base (1300) from each other,
wherein the output base (1300) is configured to cooperate with the guide transmission (1200) so that when the input base (1100) is driven by the drive mechanism (2000) to rotate, the guide transmission (1200) causes the output base (1300) to rotate in synchronization with the input base (1100),
wherein the third end of the output base (1300) is an open end, and the output base (1300) defines a cavity in which the second end of the input base (1100) is inserted,
wherein the guide transmission (1200) is defined by an external extension of the outer circumferential surface of the input base (1100), and an inner wall of the cavity is provided with a mating internal groove (1310) that matches the guide transmission (1200), **characterized in that**:

the axial limiting assembly comprises a first axial limiting sub-assembly, the first axial limiting sub-assembly comprising a third limiting stopper (1515) and a third stopper (1514), the third limiting stopper (1515) provided on the outer circumferential surface of the input base (1100), the third stopper (1514) provided on an end face of the output base (1300) at the third end, wherein the cavity of the output base (1300) is provided with a receptacle channel which matches the third limiting stopper (1515) so as to allow axial movement of the third limiting stopper (1515), and
upon the third stopper (1514) coming into interaction with the third limiting stopper (1515), the output base (1300) is stopped from further axially moving away from the input base (1100).

2. The transmission assembly (1000) according to claim 1, wherein the mating internal groove (1310) and the guide transmission (1200) cooperate with each other to define a torque transmission surface (1360) including a proximal end and a distal end, the proximal end of the torque transmission surface (1360) spaced apart from a center of the output base (1300) by a distance $r_t$ satisfying the relationship $0.3R_S \leq r_t < R_S$, the distal end of the torque transmission surface (1360) spaced apart from the center of the output base (1300) by a distance $r_m$ satisfying the relationship $r_t < r_m \leq R_S$, and wherein $(r_m - r_t) \in [0.03R_S, 0.7R_S]$, where $R_S$ represents a distance between the center and the most distant boundary of the output base (1300) from the center; and/or
wherein the transmission assembly (1000) further comprises a rolling member disposed between the guide transmission (1200) and the mating internal groove (1310) in order to enable torque transmission between the guide transmission (1200) and the mating internal groove (1310); optionally wherein the rolling member comprises rolling elements and a guide channel, which are separately provided on the guide transmission (1200) and the mating internal groove (1310), the rolling elements including at least one roller and a roller cage, each roller provided in the roller cage such as to rollable in the guide channel, the roller cage configured to keep each roller at a constant position relative to the roller cage, the guide channel arranged along an axis of the transmission assembly (1000).

3. The transmission assembly (1000) according to claim 1, wherein the guide transmission (1200) is connected to the outer circumferential surface of the input base (1100) by a cantilever, and an outer wall of the output base (1300) is provided with a mating external groove (1320) that matches the guide transmission (1200);

optionally wherein:

(i) the mating external groove (1320) and the guide transmission (1200) cooperate with each other to define a torque transmission surface (1360) including a proximal end and a distal end, the proximal end of the torque transmission surface (1360) spaced apart from a center of the output base (1300) by a distance $r_t$ satisfying the relationship $0.3R_S \leq r_t < R_S$, the distal end of the torque transmission surface (1360) spaced apart from the center of the output base (1300) by a distance $r_m$ satisfying the relationship $r_t < r_m \leq R_S$, and wherein $(r_m - rt) \in [0.03R_S, 0.7R_S]$, where Rs represents a distance between the center and the most distant boundary of the output base (1300) from the center; or

(ii) wherein the transmission assembly (1000) further comprises a rolling member disposed between the guide transmission (1200) and the mating external groove (1320) in order to enable torque transmission between the guide transmission (1200) and the mating external groove (1320);

further optionally wherein the rolling member comprises rolling elements and a guide channel, which are separately provided on the guide transmission (1200) and the mating external groove (1320), the rolling elements including at least one roller and a roller cage, each roller provided in the roller cage such as to rollable in the guide channel, the roller cage configured to keep each roller at a constant position relative to the roller cage, the guide channel arranged along an axis of the transmission assembly (1000).

4. The transmission assembly (1000) according to any one of claims 1 to 3, wherein the first end of the input base (1100) is provided with a mounting hole (1120) for enabling the input base (1100) to be coupled to the drive mechanism (2000);

optionally wherein the input base (1100) is further provided with a locking hole (1130) which cooperates with a locking element (1700) to secure the drive mechanism (2000) to the input base (1100).

5. The transmission assembly (1000) according to claim 1, wherein the axial energy storage member (1400) comprises at least one elastic element brought into abutment against the input base (1100) at one end and against the output base (1300) at the other end; and/or

wherein the axial energy storage member (1400) comprises two mutually repulsive magnetic elements, one of which is provided on the input base (1100), and the other is provided on the output base (1300).

6. The transmission assembly (1000) according to claim 1, wherein the third stopper (1514) is a second screw and the third limiting stopper (1515) is provided on the outer circumferential surface of the input base (1100) proximal to the second end such as to radially extend outwardly from the input base (1100); and/or

wherein the axial limiting assembly comprises a second axial limiting sub-assembly, the second axial limiting sub-assembly comprising a fourth stopper and a fourth limiting stopper, the fourth stopper provided on an end face of the input base (1100) at the second end, the fourth limiting stopper provided on an inner wall of the output base (1300) at the fourth end, and

wherein upon the fourth stopper coming into interaction with the fourth limiting stopper, the output base (1300) is stopped from further axially moving toward the input base (1100).

7. The transmission assembly (1000) according to claim 1, wherein the third stopper (1514) comprises a limiting end and a connecting end, the output base (1300) being provided with a connecting hole configured for threaded connection with the connecting end, and wherein when projected axially, the sum of a diameter of the connecting hole and a preset safe thickness for a threaded wall in the connecting hole is 0.6-0.8 times a diameter of the limiting end;

optionally wherein:

(i) the mating internal groove (1310) defines a line of symmetry,

wherein when projected axially, the transmission assembly (1000) defines a first radial line and a second radial line, the first radial line corresponding to the line of symmetry of the mating internal groove (1310), the second radial line being a straight line passing through a center of the transmission assembly (1000) and a start position of the third limiting stopper (1515) on the outer circumferential surface of the input base (1100) at the second end, and

wherein the start position of the third limiting stopper (1515) is within the following range:

$$\beta \in [\pi - \arcsin\frac{1.6d_2}{d_1 - d_2} - \arcsin\frac{d_2 + w}{d_1 - d_2}, \pi - \arcsin\frac{1.1d_2}{d_1 - d_2} - \arcsin\frac{d_2 + w}{d_1 - d_2}]$$ ,

where w is the sum of a width of the mating internal groove (1310) and widths of two side walls of the mating internal groove (1310), $\beta$ is an angle required for the first radial line to clockwise rotate to the second radial line, $d_1$ is a diameter of a circumscribed circle of the transmission assembly (1000), and $d_2$ is a diameter of the limiting end; or

(ii) when projected axially, a distance from any point on the third limiting stopper (1515) to a center of the limiting end is greater than 0.3 times the diameter of the limiting end.

further optionally wherein the input base (1100) is provided with a locking hole (1130) configured for connection with a drive mechanism (2000), wherein when projected axially, the locking hole (1130) defines an imaginary rectangle and the transmission assembly (1000) further defines a fourth radial line passing through the center of the transmission assembly (1000) and a middle point of a short side of the imaginary rectangle, and wherein a relative position of the locking hole (1130) is within the following range:

$$\gamma \in [\arctan\frac{L_1}{L_2} + \arcsin\frac{w}{\sqrt{L_1^2 + L_2^2}}, \pi - \arcsin\frac{1.1d_2}{d_1 - d_2} - \arcsin\frac{d_2 + w}{d_1 - d_2} - \arctan\frac{L_1}{L_2}] ,$$

where $\gamma$ is an angle required for the first radial line to clockwise rotate to the fourth radial line, $L_1$ is a length of the short side of the imaginary rectangle, and $L_2$ is a length of a long side of the imaginary rectangle.

8. A drive box (100), comprising:

a housing (3000) provided therein with at least one output hole (3100);
at least one transmission assembly (1000) according to any one of claims 1 to 7; and
at least one drive mechanism (2000) disposed in the housing (3000) so as to extend out from the output hole (3100) and come into connection with the first end of the input base (1100) in the transmission assembly (1000).

9. The drive box (100) according to claim 8, wherein the input base (1100) is provided at the first end with a mounting hole (1120) configured to enable the connection of the input base (1100) and the drive mechanism (2000), wherein the input base (1100) is provided with a first locking hole section and the drive mechanism (2000) is provided with a second locking hole section, and wherein a locking element (1700) cooperates with the first and second locking hole sections to secure the input base (1100) to the drive mechanism (2000); and/or
wherein the drive mechanism (2000) has a profiled output end portion and the mounting hole (1120) in the transmission assembly (1000) has a shape matching the profiled output end portion.

10. A surgical instrument system, comprising:

a surgical instrument comprising a slave box (200), a transmission and an instrument end assembly,
the slave box (200) provided therein with at least one slave component configured to control movement of the instrument end assembly by means of the transmission; and
the drive box (100) according to claim 8 or 9, the transmission assembly (1000) in the drive box (100) configured to be coupled to the slave component to drive the slave component and hence the instrument end assembly to move.

11. The surgical instrument system according to claim 10, further comprising:

a sterile adaptor (400) detachably connected to the slave component and the transmission assembly (1000), the sterile adaptor (400) configured to transmit kinetic power from the drive box (100) to the slave box (200); optionally wherein the sterile adaptor (400) comprises at least one intermediate rotary disc (420), wherein the output base (1300) is provided at the fourth end with a master connector (1330) and the intermediate rotary disc (420) is provided with a slave interface on a surface facing the drive box (100), wherein one of the master connector (1330) and the slave interface is an insertion recess and the other one is an insertion block, and wherein inserting the insertion block into the insertion recess results in connection of the output base (1300) with the intermediate rotary disc (420).
further optionally wherein:

(i) at least two of the master connectors (1330) are provided so as to be scattered in a non-centrosymmetric manner at the fourth end of the output base (1300), and the same number of slave connectors (430) as the number of master connectors (1330) are provided in correspondence with the respective master connectors (1330); or

(ii) each of the master connector (1330) and the slave interface defines a draft angle; or

(iii) each of the master connector (1330) and the slave interface has a sector-shaped cross section.

**Patentansprüche**

1. Übertragungsanordnung (1000) für ein chirurgisches Robotersystem, umfassend:

eine Eingangsbasis (1100) mit einem ersten Ende und einem gegenüberliegenden zweiten Ende, wobei das erste Ende zur Verbindung mit einem Antriebsmechanismus (2000) ausgebildet ist;

eine Führungsübertragung (1200), die auf einer Außenumfangsfläche der Eingangsbasis (1100) angeordnet ist;

eine Ausgangsbasis (1300) mit einem dritten Ende und einem gegenüberliegenden vierten Ende, wobei das dritte Ende näher an dem ersten Ende liegt als das vierte Ende;

ein axiales Energiespeicherelement (1400), das ausgebildet ist, Energie während einer Bewegung der Eingangsbasis und der Ausgangsbasis (1300) aufeinander zu unter der Wirkung einer externen Kraft zu speichern und bei Wegnahme der externen Kraft die Energie freizugeben, um eine Bewegung der Eingangsbasis (1100) der Ausgangsbasis (1300) voneinander weg anzutreiben;

eine axiale Begrenzungsanordnung, die ausgebildet ist, eine maximale Verschiebung der Eingangsbasis (1100) und der Ausgangsbasis (1300) voneinander zu begrenzen,

wobei die Ausgangsbasis (1300) ausgebildet ist, mit der Führungsübertragung (1200) zusammenzuwirken, so dass, wenn die Eingangsbasis (1100) durch den Antriebsmechanismus (2000) zur Rotation angetrieben wird, die Führungsübertragung (1200) bewirkt, dass die Ausgangsbasis (1300) synchron mit der Eingangsbasis (1100) rotiert,

wobei das dritte Ende der Ausgangsbasis (1300) ein offenes Ende ist, und wobei die Ausgangsbasis (1300) einen Hohlraum definiert, in welchen das zweite Ende der Eingangsbasis (1100) eingeführt wird,

wobei die Führungsübertragung (1200) durch eine externe Verlängerung der Außenumfangsfläche der Eingangsbasis (1100) definiert ist, und wobei eine Innenwand des Hohlraums mit einer Gegeninnennut (1310) versehen ist, die zu der Führungsübertragung (1200) passt, **dadurch gekennzeichnet, dass**:

die axiale Begrenzungsanordnung eine erste axiale Begrenzungsunteranordnung umfasst, wobei die erste axiale Begrenzungsunteranordnung einen dritten Begrenzungsstopper (1515) und einen dritten Stopper (1514) umfasst, wobei der dritte Begrenzungstopper (1515) an der Außenumfangsfläche der Eingangsbasis (1100) vorgesehen ist, wobei der dritte Stopper (1514) an einer Endfläche der Ausgangsbasis (1300) an dem dritten Ende vorgesehen ist, wobei der Hohlraum der Ausgangsbasis (1300) mit einem Aufnahmekanal versehen ist, der zu dem dritten Begrenzungsstopper (1515) passt, um eine axiale Bewegung des dritten Begrenzungsstoppers (1515) zu ermöglichen, und

wobei bei einer Interaktion des dritten Stoppers (1514) mit dem dritten Begrenzungsstopper (1515) die Ausgangsbasis (1300) daran gehindert wird, sich weiter axial von der Eingangsbasis (1100) wegzubewegen.

2. Übertragungsanordnung (1000) nach Anspruch 1, wobei die Gegeninnennut (1310) und die Führungsübertragung (1200) zusammenwirken, um eine Drehmomentübertragungsfläche (1360) mit einem proximalen Ende und einem distalen Ende zu definieren, wobei das proximale Ende der Drehmomentübertragungsfläche (1360) von einem Zentrum der Ausgangsbasis (1300) um einen Abstand $r_t$ beabstandet ist, der die Beziehung $0{,}3R_S \leq r_t < R_S$ erfüllt, wobei das distale Ende der Drehmomentübertragungsfläche (1360) von dem Zentrum der Ausgangsbasis (1300) um einen Abstand $r_m$ beabstandet ist, der die Beziehung $r_t < r_m \leq R_S$ erfüllt, und wobei $(r_m - r_t) \in [0{,}03R_S, 0{,}7R_S]$ ist, wobei $R_S$ einen Abstand zwischen dem Zentrum und der am weitesten entfernten Grenze der Ausgangsbasis (1300) von dem Zentrum darstellt; und/oder

wobei die Übertragungsanordnung (1000) ferner ein Rollbauteil umfasst, das zwischen der Führungsübertragung (1200) und der Gegeninnennut (1310) angeordnet ist, um eine Drehmomentübertragung zwischen der Führungsübertragung (1200) und der Gegeninnennut (1310) zu ermöglichen; wobei optional das Rollbauteil Rollelemente und einen Führungskanal umfasst, die separat an der Führungsübertragung (1200) und der Gegeninnennut (1310) vorgesehen sind, wobei die Rollelemente mindestens eine Rolle und einen Rollenkäfig umfassen, wobei jede Rolle so in dem Rollenkäfig vorgesehen ist, dass diese in dem Führungskanal rollbar ist, wobei der Rollenkäfig ausgebildet ist, jede Rolle in einer konstanten Position relativ zu dem Rollenkäfig zu halten, wobei der Führungskanal entlang einer Achse der Übertragungsanordnung (1000) angeordnet ist.

3.  Übertragungsanordnung (1000) nach Anspruch 1, wobei die Führungsübertragung (1200) mit der Außenumfangsfläche der Eingangsbasis (1100) über einen Ausleger verbunden ist, und wobei eine Außenwand der Ausgangsbasis (1300) mit einer Gegenaußennut (1320) versehen ist, die zur Führungsübertragung (1200) passt;

    wobei optional:

         (i) die Gegenaußennut (1320) und die Führungsübertragung (1200) zusammenwirken, um eine Drehmomentübertragungsfläche (1360) mit einem proximalen Ende und einem distalen Ende zu definieren, wobei das proximale Ende der Drehmomentübertragungsfläche (1360) von einem Zentrum der Ausgangsbasis (1300) um einen Abstand $r_t$ beabstandet ist, der die Beziehung $0,3R_S \le r_t < R_S$ erfüllt, wobei das distale Ende der Drehmomentübertragungsfläche (1360) von dem Zentrum der Ausgangsbasis (1300) um einen Abstand $r_m$ beabstandet ist, der die Beziehung $r_t < r_m \le R_S$ erfüllt, und wobei $(r_m - r_t) \in [0,03Rs, 0,7Rs]$ ist, wobei Rs einen Abstand zwischen dem Zentrum und der am weitesten entfernten Grenze der Ausgangsbasis (1300) von dem Zentrum darstellt; oder
         (ii) wobei die Übertragungsanordnung (1000) ferner ein Rollbauteil umfasst, das zwischen der Führungsübertragung (1200) und der Gegenaußennut (1320) angeordnet ist, um eine Drehmomentübertragung zwischen der Führungsübertragung (1200) und der Gegenaußennut (1320) zu ermöglichen;

    wobei ferner optional das Rollbauteil Rollelemente und einen Führungskanal umfasst, die separat an der Führungsübertragung (1200) und der Gegenaußennut (1320) vorgesehen sind, wobei die Rollelemente mindestens eine Rolle und einen Rollenkäfig umfassen, wobei jede Rolle so in dem Rollenkäfig vorgesehen ist, dass diese in dem Führungskanal rollbar ist, wobei der Rollenkäfig ausgebildet ist, jede Rolle in einer konstanten Position relativ zu dem Rollenkäfig zu halten, wobei der Führungskanal entlang einer Achse der Übertragungsanordnung (1000) angeordnet ist.

4.  Übertragungsanordnung (1000) nach einem der Ansprüche 1 bis 3, wobei das erste Ende der Eingangsbasis (1100) mit einem Befestigungsloch (1120) versehen ist, um es der Eingangsbasis (1100) zu ermöglichen, mit dem Antriebsmechanismus (2000) gekoppelt zu werden;
    wobei optional die Eingangsbasis (1100) ferner mit einem Verriegelungsloch (1130) versehen ist, das mit einem Verriegelungselement (1700) zusammenwirkt, um den Antriebsmechanismus (2000) an der Eingangsbasis (1100) zu befestigen.

5.  Übertragungsanordnung (1000) nach Anspruch 1, wobei das axiale Energiespeicherelement (1400) mindestens ein elastisches Element umfasst, das zum Anliegen an der Eingangsbasis (1100) an einem Ende und an der Ausgangsbasis (1300) an dem anderen Ende gebracht wird; und/oder
    wobei das axiale Energiespeicherelement (1400) zwei sich gegenseitig abstoßende magnetische Elemente umfasst, von denen eines auf der Eingangsbasis (1100) und das andere auf der Ausgangsbasis (1300) vorgesehen ist.

6.  Übertragungsanordnung (1000) nach Anspruch 1, wobei der dritte Stopper (1514) eine zweite Schraube ist und der dritte Begrenzungsstopper (1515) an der äußeren Umfangsfläche der Eingangsbasis (1100) in der Nähe des zweiten Endes vorgesehen ist, so dass er sich von der Eingangsbasis (1100) radial nach außen erstreckt; und/oder

    wobei die axiale Begrenzungsanordnung eine zweite axiale Begrenzungsunteranordnung umfasst, wobei die zweite axiale Begrenzungsunteranordnung einen vierten Stopper und einen vierten Begrenzungsstopper umfasst, wobei der vierte Stopper an einer Endfläche der Eingangsbasis (1100) an dem zweiten Ende vorgesehen ist, wobei der vierte Begrenzungsstopper an einer Innenwand der Ausgangsbasis (1300) an dem vierten Ende vorgesehen ist, und
    wobei bei einer Interaktion des vierten Stoppers mit dem vierten Begrenzungsstopper die Ausgangsbasis (1300) daran gehindert wird, sich weiter axial auf die Eingangsbasis (1100) hinzubewegen.

7.  Übertragungsanordnung (1000) nach Anspruch 1, wobei der dritte Stopper (1514) ein Begrenzungsende und ein Verbindungsende umfasst, wobei die Ausgangsbasis (1300) mit einem Verbindungsloch versehen ist und zur Gewindeverbindung mit dem Verbindungsende ausgebildet ist, und wobei bei axialer Projektion die Summe aus einem Durchmesser des Verbindungslochs und einer voreingestellten sicheren Dicke für eine Gewindewand in dem Verbindungsloch das 0,6 bis 0,8-fache eines Durchmessers des Begrenzungsendes beträgt;

wobei optional:

(i) die Gegeninnennut (1310) eine Symmetrielinie definiert,

wobei die Übertragungsanordnung (1000) bei axialer Projektion eine erste Radiallinie und eine zweite Radiallinie definiert, wobei die erste Radiallinie der Symmetrielinie der Gegeninnennut (1310) entspricht, wobei die zweite radiale Linie eine gerade Linie ist, die durch ein Zentrum der Übertragungsanordnung (1000) und eine Startposition des dritten Begrenzungsstoppers (1515) auf der Außenumfangsfläche der Eingangsbasis (1100) an dem zweiten Ende verläuft, und
wobei die Startposition des dritten Begrenzungsstoppers (1515) innerhalb des folgenden Bereichs liegt:

$$\beta \in [\pi - \arcsin \frac{1.6d_2}{d_1 - d_2} - \arcsin \frac{d_2 + w}{d_1 - d_2}, \pi - \arcsin \frac{1.1d_2}{d_1 - d_2} - \arcsin \frac{d_2 + w}{d_1 - d_2}]$$ ,

wobei w die Summe einer Breite der Gegeninnennut (1310) und Breiten von zwei Seitenwänden der Gegeninnennut (1310) ist, $\beta$ ein Winkel ist, der erforderlich ist, damit die erste radiale Linie im Uhrzeigersinn zur zweiten radialen Linie rotiert, $d_1$ ein Durchmesser eines umgelaufenen Kreises der Übertragungsanordnung (1000) ist, und $d_2$ ein Durchmesser des Begrenzungsendes ist; oder

(ii) wobei bei axialer Projektion ein Abstand von einem beliebigen Punkt auf dem dritten Begrenzungsstopper (1515) zu einer Mitte des Begrenzungsendes größer als das 0,3-fache des Durchmessers des Begrenzungsendes ist,

wobei ferner optional die Eingangsbasis (1100) mit einem Verriegelungsloch (1130) versehen ist, das zur Verbindung mit einem Antriebsmechanismus (2000) ausgebildet ist, wobei bei axialer Projektion das Verriegelungsloch (1130) ein imaginäres Rechteck definiert und die Übertragungsanordnung (1000) ferner eine vierte radiale Linie definiert, die durch die Mitte der Übertragungsanordnung (1000) und einen Mittelpunkt einer kurzen Seite des imaginären Rechtecks verläuft, und
wobei eine relative Position des Verriegelungslochs (1130) innerhalb des folgenden Bereichs liegt:

$$\gamma \in [\arctan \frac{L_1}{L_2} + \arcsin \frac{w}{\sqrt{L_1^2 + L_2^2}}, \pi - \arcsin \frac{1.1d_2}{d_1 - d_2} - \arcsin \frac{d_2 + w}{d_1 - d_2} - \arctan \frac{L_1}{L_2}]$$ ,

wobei $\gamma$ ein Winkel ist, der erforderlich ist, damit die erste radiale Linie im Uhrzeigersinn zur vierten radialen Linie rotiert, $L_1$ eine Länge der kurzen Seite des imaginären Rechtecks ist, und $L_2$ eine Länge einer langen Seite des imaginären Rechtecks ist.

8. Antriebskasten (100), umfassend:

ein darin vorgesehenes Gehäuse (3000) mit mindestens einem Ausgangsloch (3100);
mindestens eine Übertragungsanordnung (1000) nach einem der Ansprüche 1 bis 7; und
mindestens einen Antriebsmechanismus (2000), der im Gehäuse (3000) so angeordnet ist, dass er sich aus dem Ausgangsloch (3100) erstreckt und mit dem ersten Ende der Eingangsbasis (1100) in der Übertragungsanordnung (1000) in Verbindung kommt.

9. Antriebskasten (100) nach Anspruch 8, wobei die Eingangsbasis (1100) an dem ersten Ende mit einem Befestigungsloch (1120) versehen ist, das ausgebildet ist, die Verbindung der Eingangsbasis (1100) und des Antriebsmechanismus (2000) zu ermöglichen, wobei die Eingangsbasis (1100) mit einem ersten Verriegelungslochteil versehen ist und der Antriebsmechanismus (2000) mit einem zweiten Verriegelungslochteil versehen ist, und wobei ein Verriegelungselement (1700) mit dem ersten und zweiten Verriegelungslochteil zusammenwirkt, um die Eingangsbasis (1100) an dem Antriebsmechanismus (2000) zu befestigen; und/oder
wobei der Antriebsmechanismus (2000) einen profilierten Ausgangsendteil aufweist und das Befestigungsloch (1120) in der Übertragungsanordnung (1000) eine Form aufweist, die dem profilierten Ausgangsendabschnitt entspricht.

**10.** Chirurgisches Instrumentensystem, umfassend:

ein chirurgisches Instrument, umfassend einen Slave-Kasten (200), eine Übertragung und eine Instrumentenanordnung,
wobei der Slave-Kasten (200) darin mit mindestens einer Slave-Komponente ausgestattet ist, die ausgebildet ist, eine Bewegung der Instrumentenanordnung mittels der Übertragung zu steuern; und
den Antriebskasten (100) nach Anspruch 8 oder 9, wobei die Übertragungsanordnung (1000) in dem Antriebskasten (100) ausgebildet ist, mit der Slave-Komponente gekoppelt zu werden, um die Slave-Komponente anzutreiben und damit die Instrumentenanordnung zu bewegen.

**11.** Chirurgisches Instrumentensystem nach Anspruch 10, ferner umfassend:

einen steriler Adapter (400), der lösbar mit der Slave-Komponente und der Übertragungsanordnung (1000) verbunden ist, wobei der sterile Adapter (400) ausgebildet ist, kinetische Kraft von dem Antriebskasten (100) auf den Slave-Kasten (200) zu übertragen;
wobei optional der sterile Adapter (400) mindestens eine Zwischenrotationsscheibe (420) umfasst, wobei die Ausgangsbasis (1300) an dem vierten Ende mit einem Master-Anschluss (1330) und die Zwischenrotationsscheibe (420) mit einer Slave-Schnittstelle auf einer dem Antriebskasten (100) zugewandten Oberfläche versehen ist, wobei einer des Master-Anschlusses (1330) oder der Slave-Schnittstelle eine Einsteckaussparung ist und der andere ein Einsteckblock ist, und wobei ein Einstecken des Einsteckblocks in die Einsteckaussparung eine Verbindung der Ausgangsbasis (1300) mit der Zwischenrotationsscheibe (420) ergibt,
wobei ferner optional:

(i) mindestens zwei der Master-Anschlüsse (1330) so vorgesehen sind, dass diese in einer nicht zentrosymmetrischen Weise an dem vierten Ende der Ausgangsbasis (1300) verstreut sind, und die gleiche Anzahl von Slave-Anschlüssen (430) wie die Anzahl von Master-Anschlüssen (1330) in Übereinstimmung mit den jeweiligen Master-Anschlüssen (1330) bereitgestellt werden; oder
(ii) jeder der Master-Anschlüsse (1330) und die Slave-Schnittstelle eine Entformungsschräge definiert; oder
(iii) jeder der Master-Anschlüsse (1330) und die Slave-Schnittstelle einen sektorförmigen Querschnitt aufweisen.

**Revendications**

**1.** Ensemble de transmission (1000) pour un système robotique chirurgical, comprenant :

une base d'entrée (1100) ayant une première extrémité et une seconde extrémité opposée, la première extrémité étant configurée pour être connectée à un mécanisme d'entraînement (2000) ;
une transmission de guidage (1200) disposée sur une surface circonférentielle externe de la base d'entrée (1100) ;
une base de sortie (1300) ayant une troisième extrémité et une quatrième extrémité opposée, la troisième extrémité étant située plus près de la première extrémité que de la quatrième extrémité ;
un élément de stockage d'énergie axial (1400) configuré pour stocker de l'énergie pendant le mouvement de la base d'entrée et de la base de sortie (1300) l'une vers l'autre sous l'action d'une force externe et pour libérer, lors du retrait de la force externe, l'énergie pour entraîner le mouvement de la base d'entrée (1100) et la base de sortie (1300) éloignées l'une de l'autre ;
un ensemble de limitation axiale configuré pour limiter un déplacement maximum de la base d'entrée (1100) et de la base de sortie (1300) l'une par rapport à l'autre,
dans lequel la base de sortie (1300) est configuré pour coopérer avec la transmission de guidage (1200) de sorte que lorsque la base d'entrée (1100) est entraînée en rotation par le mécanisme d'entraînement (2000), la transmission de guidage (1200) amène la base de sortie (1300) à tourner en synchronisation avec le base d'entrée (1100),
dans lequel la troisième extrémité de la base de sortie (1300) est une extrémité ouverte, et la base de sortie (1300) définit une cavité dans laquelle la deuxième extrémité de la base d'entrée (1100) est insérée,
où la transmission de guidage (1200) est définie par une extension externe de la surface circonférentielle externe de la base d'entrée (1100), et une paroi interne de la cavité est dotée d'une rainure interne correspondante (1310) qui correspond à la transmission de guidage (1200), **caractérisé en ce que** :

l' ensemble de limitation axiale comprend un premier sous-ensemble de limitation axiale, le premier sous-ensemble de limitation axiale comprenant une troisième butée de limitation (1515) et une troisième butée (1514), la troisième butée de limitation (1515) étant prévue sur la surface circonférentielle externe de la base d'entrée (1100), la troisième butée (1514) étant prévue sur une face d'extrémité de la base de sortie (1300) au niveau de la troisième extrémité, la cavité de la base de sortie (1300) est dotée d'un canal réceptacle qui correspond à la troisième butée de limitation (1515) de manière à permettre un mouvement axial de la troisième butée de limitation (1515), et

lorsque la troisième butée (1514) entre en interaction avec la troisième butée de limitation (1515), la base de sortie (1300) est empêchée de s'éloigner davantage axialement de la base d'entrée (1100).

2. Ensemble de transmission (1000) selon la revendication 1, dans lequel la rainure interne correspondante (1310) et la transmission de guidage (1200) coopèrent l'une avec l'autre pour définir une surface de transmission de couple (1360) comprenant une extrémité proximale et une extrémité distale, l' extrémité proximale de la surface de transmission de couple (1360) espacé d'un centre de la base de sortie (1300) d'une distance $r_t$ satisfaire la relation $0,3R_S \leq r_t < R_S$, l' extrémité distale de la surface de transmission du couple (1360) espacé du centre de la base de sortie (1300) d'une distance $r_m$ satisfaire la relation $r_t < r_m \leq R_S$, et dans lequel $(r_m - r_t) \in [0,03R_S, 0,7R_S]$, où $R_S$ représente une distance entre le centre et la limite la plus éloignée de la base de sortie (1300) du centre; et/ou lequel Ensemble de transmission (1000) comprend en outre un élément roulant disposé entre la transmission de guidage (1200) et la rainure interne correspondante (1310) afin de permettre la transmission du couple entre la transmission de guidage (1200) et la rainure interne correspondante (13 h 10); facultativement, l' élément roulant comprend des éléments roulants et un canal de guidage, qui sont disposés séparément sur la transmission de guidage (1200) et la rainure interne correspondante (1310), les éléments roulants comprenant au moins un rouleau et une cage à rouleaux, chaque rouleau étant prévu dans la cage à rouleaux de manière à pouvoir rouler dans le canal de guidage, la cage à rouleaux étant configurée pour maintenir chaque rouleau dans une position constante par rapport au cage à rouleaux, le canal de guidage étant disposé le long d'un axe de Ensemble de transmission (1000).

3. Ensemble de transmission (1000) selon la revendication 1, dans lequel la transmission de guidage (1200) est reliée à la surface circonférentielle externe de la base d'entrée (1100) par un porte-à-faux, et un mur extérieur de la base de sortie (1300) est dotée d'une rainure externe correspondante (1320) qui correspond à la transmission de guidage (1200) ;

éventuellement où :

(i) la rainure externe correspondante (1320) et la transmission de guidage (1200) coopèrent l'une avec l'autre pour définir une surface de transmission de couple (1360) comprenant une extrémité proximale et une extrémité distale, l' extrémité proximale de la surface de transmission de couple (1360) espacé d'un centre de la base de sortie (1300) d'une distance $r_t$ satisfaire la relation $0,3R_S : \leq r_t < R_S$, l' extrémité distale de la surface de transmission du couple (1360) espacé du centre de la base de sortie (1300) d'une distance $r_m$ satisfaire la relation $r_t < r_m \leq R_S$, et dans lequel $(r_m - r_t) \in [0,03R_S, 0,7R_S]$, où $R_S$ représente une distance entre le centre et la limite la plus éloignée de la base de sortie (1300) du centre; ou (ii) dans lequel Ensemble de transmission (1000) comprend en outre un élément roulant disposé entre la transmission de guidage (1200) et la rainure externe correspondante (1320) afin de permettre la transmission du couple entre la transmission de guidage (1200) et la rainure externe correspondante (1320);

en outre, facultativement, l' élément roulant comprend des éléments roulants et un canal de guidage, qui sont disposés séparément sur la transmission de guidage (1200) et la rainure externe correspondante (1320), les éléments roulants comprenant au moins un rouleau et une cage à rouleaux, chaque rouleau étant prévu dans la cage à rouleaux de manière à pouvoir rouler dans le canal de guidage, la cage à rouleaux étant configurée pour maintenir chaque rouleau dans une position constante par rapport au cage à rouleaux, le canal de guidage étant disposé le long d'un axe de Ensemble de transmission (1000).

4. Ensemble de transmission (1000) selon l'une quelconque des revendications 1 à 3, dans lequel la première extrémité de la base d'entrée (1100) est dotée d'un trou de montage (1120) pour permettre à la base d'entrée (1100) d'être couplée au mécanisme d'entraînement (2000) ; facultativement, la base d'entrée (1100) est en outre dotée d'un trou de verrouillage (1130) qui coopère avec un élément de verrouillage (1700) pour fixer le mécanisme d'entraînement (2000) à la base d'entrée (1100).

**5.** Ensemble de transmission (1000) selon la revendication 1, dans lequel l'organe axial de stockage d'énergie (1400) comprend au moins un élément élastique amené en butée contre l'embase d'entrée (1100) à une extrémité et contre l'embase de sortie (1300) à l'autre extrémité ; et/ou

dans lequel l'élément axial de stockage d'énergie (1400) comprend deux mutuellement répulsifs des éléments magnétiques, dont l'un est disposé sur la base d'entrée (1100) et l'autre est disposé sur la base de sortie (1300).

**6.** Ensemble de transmission (1000) selon la revendication 1, dans lequel le troisième bouchon (1514) est une deuxième vis et le troisième bouchon de limitation (1515) est prévu sur la surface circonférentielle externe de la base d'entrée (1100) à proximité de la deuxième extrémité de manière à s'étendre radialement vers l'extérieur depuis la base d'entrée (1100); et/ou

dans lequel l'ensemble de limitation axiale comprend un deuxième sous-ensemble de limitation axiale, le deuxième sous-ensemble de limitation axiale comprenant une quatrième butée et une quatrième butée de limitation, la quatrième butée étant prévue sur une face d'extrémité de la base d'entrée (1100) au niveau de la deuxième butée extrémité, la quatrième butée de limitation prévue sur une paroi interne de la base de sortie (1300) à la quatrième extrémité, et

dans lequel, lorsque la quatrième butée entre en interaction avec la quatrième butée de limitation, la base de sortie (1300) est empêchée de se déplacer davantage axialement vers la base d'entrée (1100).

**7.** Ensemble de transmission (1000) selon la revendication 1, dans lequel le troisième bouchon (1514) comprend une extrémité de limitation et une extrémité de connexion, la base de sortie (1300) étant dotée d'un trou de connexion configuré pour une connexion filetée avec l'extrémité de connexion, et dans lequel, lorsqu'elle est projetée axialement, la somme d'un diamètre du trou de connexion et d'une épaisseur de sécurité prédéfinie pour une paroi filetée dans le trou de connexion est de 0,6 à 0,8 fois le diamètre de l'extrémité de limitation ;

éventuellement où :

(i) la rainure interne correspondante (1310) définit un axe de symétrie,

dans lequel, lorsqu'il est projeté axialement, l'ensemble de transmission (1000) définit une première ligne radiale et une seconde ligne radiale, la première ligne radiale correspondant à l'axe de symétrie de la rainure interne correspondante (1310), la deuxième ligne radiale étant une ligne droite passant par un centre de Ensemble de transmission (1000) et une position de départ de la troisième butée de limitation (1515) sur la surface circonférentielle externe de la base d'entrée (1100) au niveau de la deuxième fin et

dans lequel la position de départ de la troisième butée de limitation (1515) se situe dans la plage suivante :

$$\beta \in [\pi - \arcsin\frac{1.6d_2}{d_1-d_2} - \arcsin\frac{d_2+w}{d_1-d_2}, \pi - \arcsin\frac{1.1d_2}{d_1-d_2} - \arcsin\frac{d_2+w}{d_1-d_2}]$$ ,

où $w$ est la somme d'une largeur de la rainure interne correspondante (1310) et largeurs de deux parois latérales de la rainure interne correspondante (1310), $\beta$ est un angle requis pour que la première ligne radiale tourne dans le sens des aiguilles d'une montre par rapport à la deuxième ligne radiale, $d_1$ est le diamètre d'un cercle circonscrit de Ensemble de transmission (1000), et $d_2$ est le diamètre de l'extrémité limite ; ou

(ii) lorsqu'elle est projetée axialement, une distance depuis n'importe quel point sur la troisième butée de limitation (1515) jusqu'à un centre de l'extrémité de limitation est supérieure à 0,3 fois le diamètre de l'extrémité de limitation,

en outre, facultativement, la base d'entrée (1100) est dotée d'un trou de verrouillage (1130) configuré pour être connecté à un mécanisme d'entraînement (2000), dans lequel lorsqu'il est projeté axialement, le trou de verrouillage (1130) définit un rectangle imaginaire et Ensemble de transmission (1000) définit en outre une quatrième ligne radiale passant par le centre de Ensemble de transmission (1000) et le milieu d'un petit côté du rectangle imaginaire, et

dans lequel une position relative du trou de verrouillage (1130) se situe dans la plage suivante :

$$\gamma \in [\arctan \frac{L_1}{L_2} + \arcsin \frac{w}{\sqrt{L_1^2 + L_2^2}}, \pi - \arcsin \frac{1.1 d_2}{d_1 - d_2} - \arcsin \frac{d_2 + w}{d_1 - d_2} - \arctan \frac{L_1}{L_2}]$$,

où $\gamma$ est un angle requis pour que la première ligne radiale tourne dans le sens des aiguilles d'une montre jusqu'à la quatrième ligne radiale, $L_1$ est la longueur du côté court du rectangle imaginaire et $L_2$ est la longueur du côté long du rectangle imaginaire.

8. Boîtier d'entraînement (100), comprenant :

un boîtier (3000) pourvu d'au moins un trou de sortie (3100) ;
au moins un ensemble de transmission (1000) selon l'une quelconque des revendications 1 à 7; et
au moins un mécanisme d'entraînement (2000) disposé dans le boîtier (3000) de manière à s'étendre hors du trou de sortie (3100) et à entrer en connexion avec la première extrémité de la base d'entrée (1100) dans Ensemble de transmission (1000).

9. Boîter d'entraînement (100) selon la revendication 8, dans laquelle la base d'entrée (1100) est dotée à la première extrémité d'un trou de montage (1120) configuré pour permettre la connexion de la base d'entrée (1100) et du mécanisme d'entraînement (2000), la base d'entrée (1100) étant dotée d'une première section de trou de verrouillage et le mécanisme d'entraînement (2000) étant doté d'une seconde section de verrouillage section de trou, et dans lequel un élément de verrouillage (1700) coopère avec le premier et une seconde section de trou de verrouillage pour fixer la base d'entrée (1100) au mécanisme d'entraînement (2000) ; et/ou
dans lequel le mécanisme d'entraînement (2000) a une partie d'extrémité de sortie profilée et le trou de montage (1120) dans l'ensemble de transmission (1000) a une forme correspondant à la partie d'extrémité de sortie profilée.

10. Système d'instrument chirurgical, comprenant :

en tant qu'instrument chirurgical comprenant un boîtier esclave (200), une transmission et un ensemble d'extrémité d'instrument,
le boîtier esclave (200) est pourvu d' au moins un composant esclave configuré pour commander le mouvement de l'ensemble d'extrémité d'instrument au moyen de la transmission ; et
le boîtier d'entraînement (100) selon revendication 8 ou 9, Ensemble de transmission (1000) dans la boîte d'entraînement (100) configuré pour être couplé au composant esclave pour entraîner le composant esclave et donc l' ensemble d'extrémité d'instrument à se déplacer.

11. Système d'instrument chirurgical selon la revendication 10, comprenant en outre :

un adaptateur stérile (400) connecté de manière détachable au composant esclave et à l'ensemble de transmission (1000), l' adaptateur stérile (400) étant configuré pour transmettre la puissance cinétique du boîtier d'entraînement (100) au boîtier esclave (200) ;
facultativement, l'adaptateur stérile (400) comprend au moins un disque rotatif intermédiaire (420), la base de sortie (1300) étant dotée à la quatrième extrémité d'un connecteur principal (1330) et le disque rotatif intermédiaire (420) étant doté une interface esclave sur une surface faisant face au boîtier de commande (100), l'un du connecteur maître (1330) et de l' interface esclave étant un évidement d'insertion et l'autre étant un bloc d'insertion, et l'insertion du bloc d'insertion dans l' évidement d'insertion entraîne la connexion de la base de sortie (1300) avec le disque rotatif intermédiaire (420),
en outre facultativement dans lequel :

(i) au moins deux des connecteurs maîtres (1330) sont prévus de manière à être dispersés de manière non centrosymétrique au niveau de la quatrième extrémité de la base de sortie (1300), et le même nombre de connecteurs esclaves (430) le nombre de connecteurs maîtres (1330) étant prévu en correspondance avec les connecteurs maîtres respectifs (1330) ; ou
(ii) chacun des connecteurs maîtres (1330) et l' interface esclave définit un angle de dépouille ; ou
(iii) chacun des connecteurs maîtres (1330) et l' interface esclave a une forme de secteur croix section.

Fig. 1

Fig. 2a

Fig. 2b

Fig. 3a

Fig. 3b

Fig. 3c

Fig. 3d

Fig. 3e

Fig. 3f

Fig. 3g

1300

1400

1360

1200

1360

1514

Fig. 3h

$r_m$

$r_t$

0

Fig. 3i

1360

Rs

r_m

r_t

Fig. 3j

1600

1511

1340

1330

1350

1300

1513

1400

1110

1130

1200

1521

Fig. 4

Fig. 5

Fig. 6

Fig. 7

Fig. 8a

Fig. 8b

Fig. 9a

Fig. 9b

Fig. 9c

Fig. 10a

Fig. 10b

Fig. 11a

Fig. 11b

Fig. 11c

1330

1320 — 1300

1400

1110 — 1200

— 1515

— 1514

1330

1320 — 1300

— 1200

— 1100

Fig. 12a

Fig. 12b

1320

1300

1400

1515

1200

1140

1514

Fig. 12c

Fig. 13a

Fig. 13b

(a)                    (b)                    (c)

Fig. 14

Fig. 15a

Fig. 15b

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107595394 A
- CN 101340852 A
- EP 3714829 A1
- EP 3033030 A1
- US 2010170519 A1